# EUROPÄISCHE PATENTSCHRIFT

(11) **EP 0 874 908 B1**
(45) Veröffentlichungstag und Bekanntmachung des Hinweises auf die Patenterteilung: **15.10.2003**
(21) Anmeldenummer: 97901533.6
(22) Anmeldetag: 15.01.1997
(51) Int. Cl.: C12N 15/82, C12N 15/54, C12N 9/10, C12N 5/10, C08B 30/00, A01H 5/00

(54) **NUCLEINSÄUREMOLEKÜLE AUS PFLANZEN CODIEREND ENZYME, DIE AN DER STÄRKESYNTHESE BETEILIGT SIND**
NUCLEIC ACID MOLECULES FROM PLANTS ENCODING ENZYMES WHICH PARTICIPATE IN THE STARCH SYNTHESIS
MOLECULES D'ACIDE NUCLEIQUE ISSUES DE VEGETAUX CODANT POUR DES ENZYMES PARTICIPANT A LA SYNTHESE DE L'AMIDON

(30) Priorität: 16.01.1996 DE 19601365
(43) Veröffentlichungstag der Anmeldung: 04.11.1998
(73) Patentinhaber: PlantTec Biotechnologie GmbH, 14473 Potsdam (DE)
(72) Erfinder: KOSSMANN, Jens, 4000 Roskilde (DK); FROHBERG, Claus, D-12249 Berlin (DE)
(74) Vertreter: VOSSIUS & PARTNER
(86) Internationale Anmeldenummer: EP9700158
(87) Internationale Veröffentlichungsnummer: WO97026362

(56) Entgegenhaltungen:
- WO-A-94/09144
- PLANT PHYSIOLOGY, Bd. 103, Nr. 2, 1.Oktober 1993, Seiten 565-573, XP000565731 TADASHI BABA ET AL: "IDENTIFICATION, CDNA CLONING, AND GENE EXPRESSION OF SOLUBLE STARCH SYNTHASE IN RICE (ORYZA SATIVA L.) IMMATURE SEEDS" in der Anmeldung erwähnt
- THE PLANT JOURNAL, Bd. 6, Nr. 2, 1994, Seiten 151-159, XP002031374 MU C. ET AL.: "Association of a 76 kDa polypeptide with soluble starch synthase I activity in maize (cv B73) endosperm" in der Anmeldung erwähnt
- THE PLANT JOURNAL, Bd. 8, Nr. 2, August 1995, Seiten 283-294, XP002031375 EDWARDS A. ET AL.: "Biochemical and molecular characterization of a novel starch synthase from potato tubers" in der Anmeldung erwähnt
- PLANT, CELL AND ENVIRONMENT, Bd. 17, 1994, Seiten 601-613, XP002005943 MÜLLER-RÖBER B. AND KOSSMANN J.: "Approaches to influence starch quantity and starch quality in transgenic plants"

## Beschreibung

Die vorliegende Erfindung betrifft Nucleinsäuremoleküle, die ein Enzym codieren, das an der Stärkesynthese in Pflanzen beteiligt sind. Bei diesem Enzym handelt es sich um eine neue Isoform der Stärkesynthase.
Weiterhin betrifft diese Erfindung Vektoren, Bakterien, sowie mit den beschriebenen Nucleinsäuremolekülen transformierte Pflanzenzellen und aus diesen regenerierbare Pflanzen.
Ferner werden Verfahren zur Herstellung transgener Pflanzen beschrieben, die aufgrund der Einführung von DNA-Molekülen, die eine Stärkesynthase codieren, eine in ihren Eigenschaften veränderte Stärke synthetisieren.

Im Hinblick auf die zunehmende Bedeutung, die pflanzlichen Inhaltsstoffen als erneuerbaren Rohstoffquellen in letzter Zeit beigemessen wird, ist es eine der Aufgaben der biotechnologischen Forschung, sich um eine Anpassung dieser pflanzlichen Rohstoffe an die Anforderungen der verarbeitenden Industrie zu bemühen. Um eine Anwendung von nachwachsenden Rohstoffen in möglichst vielen Einsatzgebieten zu ermöglichen, ist es darüber hinaus erforderlich, eine große Stoffvielfalt zu erreichen.
Neben Ölen, Fetten und Proteinen stellen Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in höheren Pflanzen ist. Hierbei ist Mais eine der interessantesten Pflanzen, da sie die weltweit für die Stärkeproduktion wichtigste Kulturpflanze ist.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades und des Auftretens von Verzweigungen der Glucoseketten unterscheiden. Daher stellt Stärke keinen einheitlichen Rohstoff dar. Man unterscheidet insbesondere die Amylose-Stärke, ein im wesentlichen unverzweigtes Polymer aus α-1,4-glycosidisch verknüpften Glucosemolekülen, von der Amylopektin-Stärke, die ihrerseits ein komplexes Gemisch aus unterschiedlich verzweigten Glucoseketten darstellt. Die Verzweigungen kommen dabei durch das Auftreten von zusätzlichen α-1,6-glycosidischen Verknüpfungen zustande. In typischen für die Stärkeproduktion verwendeten Pflanzen, wie z.B. Mais oder Kartoffel, besteht die synthetisierte Stärke zu ca. 25 % aus Amylose-Stärke und zu ca. 75 % aus Amylopektin-Stärke.
Um eine möglichst breite Anwendung von Stärke zu ermöglichen, erscheint es wünschenswert, Pflanzen zur Verfügung zu stellen, die in der Lage sind, modifizierte Stärke zu synthetisieren, die sich für verschiedene Verwendungszwecke besonders eignet. Eine Möglichkeit, derartige Pflanzen bereitzustellen, besteht - neben züchterischen Maßnahmen - in der gezielten genetischen Veränderung des Stärkemetabolismus stärkeproduzierender Pflanzen durch gentechnologische Methoden. Voraussetzung hierfür ist jedoch die Identifizierung und Charakterisierung der an der Stärkesynthese und/oder -modifikation beteiligten Enzyme sowie die Isolierung der entsprechenden, diese Enzyme codierende DNA-Moleküle.
Die biochemischen Synthesewege, die zum Aufbau von Stärke führen, sind im wesentlichen bekannt. Die Stärkesynthese in pflanzlichen Zellen findet in den Plastiden statt. In photosynthetisch aktiven Geweben sind dies die Chloroplasten, in photosynthetisch inaktiven, stärkespeichernden Geweben die Amyloplasten.
Die wichtigsten an der Stärkesynthese beteiligten Enzyme sind die Stärkesynthasen sowie die Verzweigungsenzyme. Bei den Stärkesynthasen sind verschiedene Isoformen beschrieben, die alle eine Polymerisierungsreaktion durch Übertragung eines Glucosylrestes von ADP-Glucose auf α-1,4-Glucane katalysieren. Verzweigungsenzyme katalysieren die Einführung von α-1,6-Verzweigungen in lineare α-1,4-Glucane.
Stärkesynthasen können in zwei Klassen eingeteilt werden: die Stärkekorn-gebundenen Stärkesynthasen ("granule-bound starch synthases"; GBSS) und die löslichen Stärkesynthasen ("soluble starch synthases"; SSS). Diese Unterscheidung ist nicht in jedem Fall eindeutig zu treffen, da einige der Stärkesynthasen sowohl stärkekorngebunden als auch in löslicher Form vorliegen (Denyer et al., Plant J. 4 (1993), 191-198; Mu et al., Plant J. 6 (1994), 151-159). Für verschiedene Pflanzenspezies werden innerhalb dieser Klassen wiederum verschiedene Isoformen beschrieben, die sich hinsichtlich ihrer Abhängigkeit von Startermolekülen unterscheiden (sogenannte "primer dependent" (Typ II) und "primer independent" (Typ I) starch synthases).

Lediglich für die Isoform GBSS I gelang es bisher, die genaue Funktion bei der Stärkesynthese zu ermitteln. Pflanzen, in denen diese Enzymaktivität stark oder vollkommen reduziert ist, synthetisieren eine amylosefreie (sogenannte "waxy") Stärke (Shure et al., Cell 35 (1983), 225-233; Visser et al., Mol. Gen. Genet. 225 (1991), 289-296; WO 92/11376), so daß diesem Enzym eine entscheidende Rolle bei der Synthese der Amylose-Stärke zugesprochen wird. Dieses Phänomen wird ebenfalls in Zellen der Grünalge Chlamydomonas reinhardtii beobachtet (Delrue et al., J. Bacteriol. 174 (1992), 3612-3620). Bei Chlamydomonas konnte darüber hinaus gezeigt werden, daß GBSS I nicht nur an der Synthese der Amylose beteiligt ist, sondern auch einen Einfluß auf die Amylopektinsynthese besitzt. In Mutanten, die keine GBSS I-Aktivität aufweisen, fehlt eine bestimmte Fraktion des normalerweise synthetisierten Amylopektins, die längerkettige Glucane aufweist.

Die Funktionen der anderen Isoformen der Stärkekorn-gebundenen Stärkesynthasen, insbesondere der GBSS II, und der löslichen Stärkesynthasen sind bisher unklar. Es wird angenommen, daß die löslichen Stärkesynthasen zusammen mit Verzweigungsenzymen an der Synthese des Amylopektins beteiligt sind (siehe z.B. Ponstein et al., Plant Physiol. 92 (1990), 234-241) und daß sie eine wichtige Funktion bei der Regulation der Stärkesyntheserate spielen.

Bei Mais wurden zwei Isoformen der Stärkekorn-gebundenen, sowie zwei bzw. drei Isoformen der löslichen Stärkesynthasen identifiziert (Hawker et al., Arch. Biochem. Biophys. 160 (1974), 530-551; Pollock und Preiss, Arch. Biochem. Biophys. 204 (1980), 578-588; MacDonald und Preiss, Plant Physiol. 78 (1985), 849-852; Mu et al., Plant J. 6 (1994), 151-159).
Eine GBSS I aus Mais codierende cDNA sowie eine genomische DNA sind bereits beschrieben (Shure et al., Cell 35 (1983), 225-233; Kloesgen et al., Mol. Gen. Genet. 203 (1986), 237-244). Weiterhin ist ein sogenannter "Expressed Sequence Tag" (EST) beschrieben worden (Shen et al., 1994, GenBank Nr.: T14684), dessen abgeleitete Aminosäuresequenz eine starke Ähnlichkeit zur abgeleiteten Aminosäuresequenz der GBSS II aus Erbse (Dry et al., Plant J. 2 (1992), 193-202) und Kartoffel (Edwards et al., Plant J. 8 (1995), 283-294) aufweist. Nucleinsäuresequenzen, die weitere Stärkesynthase-Isoformen aus Mais codieren, lagen jedoch bisher noch nicht vor. cDNA-Sequenzen, die für andere Stärkesynthasen als für die GBSS I codieren, wurden bisher lediglich für Erbse (Dry et al., Plant J. 2 (1992), 193-202), Reis (Baba et al., Plant Physiol. 103 (1993), 565-573) und Kartoffel (Edwards et al., Plant J. 8 (1995), 283-294) beschrieben.
Außer beim Mais wurden lösliche Stärkesynthasen auch in einer Reihe weiterer Pflanzenarten identifiziert. Lösliche Stärkesynthasen sind beispielsweise bis zur Homogenität aus Erbse (Denyer und Smith, Planta 186 (1992), 609-617) und Kartoffel (Edwards et al., Plant J. 8 (1995), 283-294) isoliert worden. In diesen Fällen stellte-sich heraus, daß die als SSS II identifizierte Isoform der löslichen Stärkesynthase identisch ist mit der Stärkekorn-gebundenen Stärkesynthase GBSS II (Denyer et al., Plant J. 4 (1993), 191-198; Edwards et al., Plant J. 8 (1995), 283-294). Für einige weitere Pflanzenspezies wurde das Vorhandensein mehrerer SSS-Isoformen mit Hilfe chromatographischer Methoden beschrieben, beispielsweise bei Gerste (Tyynelä und Schulman, Physiologia Plantarum 89 (1993) 835-841; Kreis, Planta 148 (1980), 412-416) und Weizen (Rijven, Plant Physiol. 81 (1986), 448-453). DNA-Sequenzen, die diese Proteine codieren, wurden jedoch bisher nicht beschrieben.
Um weitere Möglichkeiten bereitzustellen, beliebige stärkespeicherde Pflanzen dahingehend zu verändern, daß sie eine modifizierte Stärke synthetisieren, ist es erforderlich, jeweils DNA-Sequenzen zu identifizieren, die weitere Isoformen- der

Stärkesynthasen codieren.

Der vorliegenden Erfindung liegt somit die Aufgabe zugrunde, Nucleinsäuremoleküle zur Verfügung zu stellen, die an der Stärkebiosynthese beteiligte Enzyme codieren und mit deren Hilfe es möglich ist, gentechnisch veränderte Pflanzen herzustellen, die eine erhöhte oder erniedrigte Aktivität dieser Enzyme aufweisen, wodurch es zu einer Veränderung der chemischen und/oder physikalischen Eigenschaften der in diesen Pflanzen synthetisierten Stärke kommt.

Diese Aufgabe wird durch die Bereitstellung der in den Patentansprüchen bezeichneten Ausführungsformen gelöst.

Die vorliegende Erfindung betrifft daher Nucleinsäuremoleküle, die Proteine mit der biologischen Aktivität einer Stärkesynthase codieren, wobei derartige Moleküle vorzugsweise Proteine codieren, die die unter Seq ID No. 2 angegebene Aminosäuresequenz umfassen. Insbesondere betrifft die Erfindung Nucleinsäuremoleküle, die die unter Seq ID No. 1 angegebene Nucleotidsequenz oder einen Teil davon enthalten, bevorzugt Moleküle, die die in Seq ID No. 1 angegebene codierende Region umfassen bzw. entsprechende Ribonucleotidsequenzen
Gegenstand der Erfindung sind ebenfalls Nucleinsäuremoleküle, die eine Stärkesynthase codieren und deren Sequenz aufgrund der Degeneration des genetischen Codes von den Nucleotidsequenzen der oben beschriebenen Moleküle abweicht. Ferner betrifft die vorliegende Erfindung Nucleinsäuremoleküle, die eine Stärkesynthase codieren und die mit einem der oben beschriebenen Moleküle unter stringenten Bedingungen hybridisieren. Die Erfindung betrifft auch Nucleinsäuremoleküle, die eine Sequenz auf weisen, die zu der gesamten oder einem Teil der Sequenz der obengenannten Nucleinsäuremoleküle komplementär ist.

Bei den erfindungsgemäßen Nucleinsäuremolekülen kann es sich sowohl um DNA- als auch RNA-Moleküle handeln. Entsprechende DNA-Moleküle sind beispielsweise genomische oder cDNA-Moleküle. RNA-Moleküle können beispielsweise mRNA oder antisense-RNA-Moleküle sein.
Der Begriff "Hybridisierung" bedeutet im.Rahmen dieser Erfindung eine Hybridisierung unter stringenten Bedingungen, wie sie beispielsweise in Sambrock et al.,- Molecular Cloning, A Laboratory Manual, 2. Aufl. (1989) Cold Spring Harbor Laboratory Press, Cold Spring Harbor, NY) beschrieben sind. Nucleinsäuremoleküle, die mit den erfindungsgemäßen Nucleinsäuremolekülen hybridisieren, können prinzipiell aus jedem beliebigen Organismus (d.h. Prokaryonten oder Eukaryonten, insbesondere aus Bakterien, Pilzen, Algen, Pflanzen oder tierischen Organismen) stammen, der derartige Moleküle besitzt. Sie stammen vorzugsweise aus monokotylen oder dikotylen Pflanzen, insbesondere aus Nutzpflanzen, und besonders bevorzugt aus stärkespeichernden Pflanzen, insbesondere aus Mais.
Nucleinsäuremoleküle, die mit den erfindungsgemäßen. Molekülen hybridisieren, können z.B. aus genomischen oder aus cDNA-Bibliotheken verschiedener Organismen isoliert werden.
Die Identifizierung und Isolierung derartiger Nucleinsäuremoleküle aus Pflanzen oder anderen Organismen kann dabei unter Verwendung der erfindungsgemäßen Moleküle oder Teile dieser Moleküle bzw. der reversen Komplemente dieser Moleküle erfolgen, z.B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY).
Als Hybridisierungsprobe können z.B. Nucleinsäuremoleküle verwendet werden, die exakt die oder im wesentlichen die unter Seq ID No. 1 angegebene Nucleotidsequenz oder Teile dieser Sequenz aufweisen. Bei den als Hybridisierungsprobe verwendeten Fragmenten kann es sich auch um synthetische Fragmente handeln, die mit Hilfe der gängigen Synthesetechniken hergestellt wurden und deren Sequenz im wesentlichen mit der-eines erfindungsgemäßen Nucleinsäuremoleküls übereinstimmt. Hat man Gene identifiziert und isoliert, die mit den erfindungsgemäßen Nucleinsäuresequenzen hybridisieren, ist eine Bestimmung der Sequenz und eine Analyse der Eigenschaften der von dieser Sequenz codierten Proteine erforderlich.

Die mit den erfindungsgemäßen Nucleinsäuremolekülen hybridisierenden Moleküle umfassen auch Fragmente, Derivate und allelische Varianten der oben beschriebenen DNA-Moleküle, die ein erfindungsgemäßes Protein codieren. Unter Fragmenten werden dabei Teile der Nucleinsäuremoleküle verstanden, die lang genug sind, um eines der beschriebenen Proteine zu codieren. Der Ausdruck Derivat bedeutet in diesem Zusammenhang, daß die Sequenzen dieser Moleküle sich von den Sequenzen der oben beschriebenen Nucleinsäuremoleküle an einer oder mehreren Positionen unterscheiden und einen hohen Grad an Homologie zu diesen Sequenzen aufweisen. Homologie bedeutet dabei eine Sequenzidentität
von mindestens 60 %, vorzugsweise über 80 % und besonders bevorzugt über 90 %. Die Abweichungen zu den oben beschriebenen Nucleinsäuremolekülen können dabei durch Deletion, Substitution, Insertion oder Rekombination entstanden sein.
Homologie bedeutet ferner, daß funktionelle und/oder strukturelle Äquivalenz zwischen den betreffenden Nucleinsäuremolekülen oder den durch sie codierten Proteinen, besteht. Bei den Nucleinsäuremolekülen, die homolog zu den oben beschriebenen Molekülen sind und Derivate dieser Moleküle darstellen, handelt es sich in der Regel um Variationen dieser Moleküle, die Modifikationen darstellen, die dieselbe biologische Funktion ausüben. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten.

Die von den verschiedenen Varianten der erfindungsgemäßen Nucleinsäuremoleküle codierten Proteine weisen bestimmte gemeinsame Charakteristika auf. Dazu können z.B. Enzymaktivität, Molekulargewicht, immunologische Reaktivität, Konformation etc. gehören, sowie physikalische Eigenschaften wie z.B. das Laufverhalten in Gelelektrophoresen, chromatographisches Verhalten, Sedimentationskoeffizienten, Löslichkeit, spektroskopische Eigenschaften, Stabilität; pH-Optimum, Temperatur-Optimum etc.
Wichtige Charakteristika einer Stärkesynthase sind: i) ihre Lokalisation im Stroma der Plastiden pflanzlicher Zellen; ii) ihre Fähigkeit zur Synthese linearer α-1,4-verknüpfter Polyglucane unter Verwendung von ADP-Glucose als Substrat. Diese Aktivität kann wie in Denyer und Smith (Planta 186 (1992), 606-617) oder wie in den Beispielen beschrieben bestimmt werden.
Die erfindungsgemäßen Nucleinsäuremoleküle können prinzipiell aus jedem Organismus stammen, der die beschriebenen Proteine exprimiert, vorzugsweise aus Pflanzen, insbesondere aus stärkesynthetisierenden bzw. stärkespeichernden Pflanzen. Diese können sowohl monokotyle oder auch dikotyle Pflanzen sein. Besonders bevorzugt sind dabei z.B. Getreidearten (wie Gerste, Roggen, Hafer, Weizen etc.), Mais, Reis, Erbse, Maniok, Kartoffel usw.
Bei den durch die erfindungsgemäßen Nucleinsäuremolekülen codierten Proteine handelt es sich um eine bisher nicht identifizierte und charakterisierte Isoform einer pflanzlichen Stärkesynthase. Diese Proteine weisen die enzymatische Aktivität einer Stärkesynthase auf, sowie auch gewisse Homologiebereiche zu bisher bekannten Stärkesynthasen aus Pflanzen, können aber keiner der bisher bekannten Isoformen eindeutig zugeordnet werden.
Die durch die erfindungsgemäßen Nucleinsäuremoleküle codierten Proteine weisen insbesondere die Eigenschaft auf, daß sie nach Einführung in eine E. coli-Mutante, bei der sämtliche glg-Gene deletiert sind und die eine mutierte, deregulierte ADP-Glucose-Pyrophosphorylase exprimiert, Kultivierung dieser Mutante auf Glucose-haltigem Medium und Jodbedampfung zur Blaufärbung der Bakterienkolonien führt.

Gegenstand der Erfindung sind auch Oligonucleotide, die spezifisch mit einem erfindungsgemäßen Nucleinsäuremolekül hybridisieren. Derartige Oligonucleotide haben vorzugsweise eine Länge von mindestens 10, insbesondere von mindestens 15 und besonders bevorzugt von mindestens 50 Nucleotiden. Diese Oligonucleotide sind dadurch gekennzeichnet, daß sie spezifisch mit erfindungsgemäßen Nucleinsäuremolekülen hybridisieren, d.h. nicht oder nur in sehr geringem Ausmaß mit Nucleinsäuresequenzen, die andere Proteine, insbesondere andere Stärkesynthasen codieren. Die erfindungsgemäßen Oligonucleotide können beispielsweise als Primer für eine PCR-Reaktion verwendet werden. Ebenso können sie Bestandteile von antisense-Konstrukten sein oder von DNA-Molekülen, die für geeignete Ribozyme codieren.

Ferner betrifft die Erfindung Vektoren, insbesondere Plasmide, Cosmide, Viren, Bacteriophagen und andere in der Gentechnik gängige Vektoren, die die oben beschriebenen erfindungsgemäßen Nucleinsäuremoleküle enthalten.

In einer bevorzugten Ausführungsform sind die in den Vektoren enthaltenen Nucleinsäuremoleküle verknüpft mit regulatorischen Elementen, die die Transkription und Synthese einer translatierbaren RNA in prokaryontischen oder eukaryontischen Zellen gewährleisten.
Die Expression der erfindungsgemäßen Nucleinsäuremoleküle in prokaryontischen Zellen, beispielsweise in Escherichia coli, ist insofern interessant, als daß auf diese Weise eine genauere Charakterisierung der enzymatischen Aktivitäten der Enzyme, für die diese Moleküle codieren, ermöglicht wird. Es ist insbesondere möglich, das Produkt, das von den entsprechenden Enzymen in Abwesenheit anderer, in der pflanzlichen Zelle an der Stärkesynthese beteiligter Enzyme synthetisiert wird, zu charakterisieren. Dies läßt Rückschlüsse zu auf die Funktion, die das entsprechende Protein bei der Stärkesynthese in der Pflanzenzelle ausübt.
Darüber hinaus ist es möglich, mittels gängiger molekularbiologischer Techniken (siehe z.B. Sambrook et al., 1989, Molecular Cloning, A Laboratory Manual, 2. Aufl. Cold Spring Harbor Laboratory Press, Cold Spring Harbor, NY) verschiedenartige Mutationen in die erfindungsgemäßen Nucleinsäuremoleküle einzuführen, wodurch es zur Synthese von Proteinen mit eventuell veränderten biologischen Eigenschaften kommt. Hierbei ist zum einen die Erzeugung von Deletionsmutanten möglich, bei denen durch fortschreitende Deletionen vom 5'- oder vom 3'- Ende der codierenden DNA-Sequenz Nucleinsäuremoleküle erzeugt werden, die zur Synthese entsprechend verkürzter Proteine führen. Durch derartige Deletionen am 5'-Ende der Nucleotidsequenz ist es beispielsweise möglich, Aminosäuresequenzen zu identifizieren, die für die Translokation des Enzyms in die Plastiden verantwortlich sind (Transitpeptide). Dies erlaubt es, gezielt Enzyme herzustellen, die durch Entfernen der entsprechenden Sequenzen nicht mehr in den Plastiden, sondern im Cytosol lokalisiert sind, oder aufgrund der Addition von andereren Signalsequenzen in anderen Kompartimenten lokalisiert sind.
Andererseits ist auch die Einführung von Punktmutationen denkbar an Positionen, bei denen eine Veränderung der Aminosäuresequenz einen Einfluß beispielweise auf die Enzymaktivität oder die Regulierung des Enzyms hat. Auf diese Weise können z.B. Mutanten hergestellt werden, die einen veränderten Kₘ-Wert besitzen oder nicht mehr den normalerweise in der Zelle vorliegenden Regulationsmechanismen über allosterische Regulation oder kovalente Modifizierung unterliegen.
Des weiteren können Mutanten hergestellt werden, die eine veränderte Substrat- oder Produktspezifität aufweisen, wie z.B. Mutanten, die als Substrat ADP-Glucose-6-Phosphat anstatt ADP-Glucose verwenden. Weiterhin können Mutanten hergestellt werden, die ein verändertes Aktivitäts-Temperatur-Profil aufweisen.
Für die gentechnische Manipulation in prokaryontischen Zellen können die erfindungsgemäßen Nucleinsäuremoleküle oder Teile dieser Moleküle in Plasmide eingebracht werden, die eine Mutagenese oder eine Sequenzveränderung durch Rekombination von DNA-Sequenzen erlauben. Mit Hilfe von Standardverfahren (vgl. Sambrook et al., 1989, Molecular Cloning: A laboratory manual, 2. Aufl., Cold Spring Harbor Laboratory Press, NY, USA) können Basenaustausche vorgenommen oder natürliche oder synthetische Sequenzen hinzugefügt werden. Für die Verbindung der DNA-Fragmente untereinander können an die Fragmente Adaptoren oder linker angesetzt werden. Ferner können Manipulationen, die passende Restriktionsschnittstellen zur Verfügung stellen oder die überflüssige DNA oder Restriktionsschnittstellen entfernen, eingesetzt werden. Wo Insertionen, Deletionen oder Substitutionen in Frage kommen, können in vitro-Mutagenese, "primer repair", Restriktion oder Ligation verwendet werden. Als Analysemethode werden im allgemeinen eine Sequenzanalyse, eine Restriktionsanalyse und weitere biochemisch-molekularbiologische Methoden durchgeführt.

In einer weiteren Ausführungsform betrifft die Erfindung Wirtszellen, insbesondere prokaryontische oder eukaryontische Zellen, die mit einem oben beschriebenen erfindungsgemäßen Nucleinsäuremolekül oder einem erfindungsgemäßen Vektor transformiert und genetisch modifiziert sind, sowie Zellen, die von derart transformierten Zellen abstammen und ein erfindungsgemäßes Nucleinsäuremolekül oder einen Vektor enthalten. Dabei handelt es sich vorzugsweise um bakterielle Zellen oder pflanzliche Zellen.

Gegenstand der Erfindung sind ferner die Proteine oder biologisch aktive Fragmente davon, die durch die erfindungsgemäßen Nucleinsäuremoleküle codiert werden, sowie Verfahren zu deren Herstellung, wobei eine erfindungsgemäße Wirtszelle unter Bedingungen kultiviert wird, die die Synthese des Proteins erlauben, und anschließend das Protein aus den kultivierten Zellen und/oder dem Kulturmedium isoliert wird.

Durch die Bereitstellung der erfindungsgemäßen Nucleinsäuremoleküle ist es nun möglich, mit Hilfe gentechnischer Methoden in den Stärkemetabolismus von Pflanzen einzugreifen, wie es bisher nicht möglich war, und ihn dahingehend zu verändern, daß es zur Synthese einer modifizierten Stärke kommt, die beispielsweise in ihren physikalisch-chemischen Eigenschaften, insbesondere dem Amylose/Amylopektin-Verhältnis, dem Verzweigungsgrad, der durchschnittlichen Kettenlänge, dem Phosphatgehalt, dem Verkleisterungsverhalten, der Stärkekorngröße und/oder der Stärkekornform im Vergleich zu in Wildtyp-Pflanzen synthetisierter Stärke verändert ist. Durch eine Erhöhung der Aktivität der erfindungsgemäßen Proteine, beispielsweise durch Uberexpression entsprechender Nucleinsäuremoleküle, oder durch die Bereitstellung von Mutanten, die nicht mehr den zelleigenen Regulationsmechanismen unterliegen und/oder unterschiedliche Temperaturabhängigkeiten in bezug auf ihre Aktivität besitzen, besteht die Möglichkeit der Ertragssteigerung in entsprechend gentechnisch veränderten Pflanzen. Die wirtschaftliche Bedeutung der Möglichkeit des Eingriffs in die Stärkesynthese allein bei Mais ist offensichtlich: Mais ist weltweit die wichtigste Pflanze zur Stärkegewinnung. Ca. 80% der weltweit jährlich produzierten Stärke wird aus Mais gewonnen.
Möglich ist somit die Expression der erfindungsgemäßen Nucleinsäuremoleküle in pflanzlichen Zellen, um die Aktivität der entsprechenden Stärkesynthase zu erhöhen. Ferner ist es möglich, die erfindungsgemäßen Nucleinsäuremoleküle nach dem Fachmann bekannten Methoden zu modifizieren, um erfindungsgemäß Stärkesynthasen zu erhalten, die nicht mehr den zelleigenen Regulationsmechanismen unterliegen, bzw. veränderte Temperaturabhängigkeiten oder Substrat- bzw. Produktspezifitäten aufweisen.
Bei der Expression der erfindungsgemäßen Nucleinsäuremoleküle in Pflanzen besteht grundsätzlich die Möglichkeit, daß das synthetisierte Protein in jedem beliebigen Kompartiment der pflanzlichen Zelle lokalisiert sein kann. Um die Lokalisation in einem bestimmten Kompartiment zu erreichen, muß die die Lokalisation in Plastiden gewährleistende Sequenz deletiert werden und die verbleibende codierende Region gegebenenfalls mit DNA-Sequenzen verknüpft werden, die die Lokalisierung in dem jeweiligen Kompartiment gewährleisten. Derartige Sequenzen sind bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die vorliegende Erfindung betrifft somit auch transgene Pflanzenzellen, die mit einem erfindungsgemäßen Nucleinsäuremolekül transformiert und genetisch modifiziert wurden, sowie transgene Pflanzenzellen, die von derartig tranformierten Zellen abstammen. Derartige Zellen enthalten ein erfindungsgemäßes Nucleinsäuremolekül, wobei dieses vorzugsweise mit regulatorischen DNA-Elementen verknüpft ist, die die Transkription in pflanzlichen Zellen gewährleisten, insbesondere mit einem Promotor. Die transgenen erfindungsgemäßen Pflanzenzellen lassen sich von natürlicherweise auftretenden Pflanzenzellen dadurch unterscheiden, daß sie integriert in ihr Genom ein erfindungsgemäßes DNA-Molekül enthalten, welches in den Zellen natürlicherweise entweder überhaupt nicht vorhanden ist, oder aber in einer anderen genetischen Umgebung, d.h. an einem anderen Ort im Genom. Pflanzenzellen, die gegebenenfalls natürlicherweise in ihrem Genom ein erfindungsgenmäßes DNA-Molekül enthalten, unterscheiden sich von erfindungsgemäßen Pflanzenzellen dadurch, daß letztere mehr Genkopien der erfindungsgemäßen Nucleinsäuremoleküle aufweisen, als natürlicherweise in den jeweiligen natürlicherweise vorkommenden Pflanzenzellen auftreten und daß diese zusätzlichen Kopien an anderen genetischen Loci integriert sind. Die oben erwähnten Merkmale lassen sich beispielsweise durch Southern Blot-Analyse genomischer DNA bestimmen.
Die transgenen Pflanzenzellen können nach dem Fachmann bekannten Techniken zu ganzen Pflanzen regeneriert werden. Die durch Regeneration der erfindungsgemäßen transgenen Pflanzenzellen erhältlichen Pflanzen sind ebenfalls Gegenstand der vorliegenden Erfindung. Ferner sind Gegenstand der Erfindung Pflanzen, die die obenbeschriebenen transgenen Pflanzenzellen enthalten. Bei den transgenen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, insbesondere um stärkesynthetisierende bzw. stärkespeichernde Pflanzen, wie z.B. Getreidearten (Roggen, Gerste Hafer, Weizen etc.), Reis, Mais, Erbse, Maniok oder Kartoffel.
Die Erfindung betrifft ebenfalls Vermehrungsmaterial der erfindungsgemäßen Pflanzen, beispielsweise Früchte, Samen, Knollen, Wurzelstöcke, Sämlinge, Stecklinge etc.

Die erfindungsgemäßen transgenen Pflanzenzellen und Pflanzen synthetisieren aufgrund der Expression bzw. zusätzlichen Expression eines erfindungsgemäßen Nucleinsäuremoleküls eine Stärke, die beispielsweise in ihren physikalisch-chemischen Eigenschaften, insbesondere dem Amylose/Amylopektin-Verhältnis, dem Verzweigungsgrad, der durchschnittlichen Kettenlänge, dem Phosphatgehalt, dem Verkleisterungsverhalten, der Stärkekorngröße und/oder der Stärkekornform im Vergleich zu in Wildtyp-Pflanzen synthetisierter Stärke verändert ist. Insbesondere kann eine solche Stärke im Hinblick auf die Viskosität und/oder die Gelbildungseigenschaften von Kleistern dieser Stärke im Vergleich zu Wildtypstärke verändert sein.
Gegenstand der vorliegenden Erfindung ist somit auch die aus den erfindungsgemäßen transgenen Pflanzenzellen und Pflanzen erhältliche Stärke.

Ferner ist es möglich, mit Hilfe der erfindungsgemäßen Nucleinsäuremoleküle Pflanzenzellen und Pflanzen zu erzeugen, bei denen die Aktivität eines erfindungsgemäßen Proteins verringert ist. Dies führt ebenfalls zur Synthese einer Stärke mit veränderten chemischen und/oder physikalischen Eigenschaften verglichen mit Stärke aus Wildtyp-Pflanzenzellen.
Ein weiterer Gegenstand der Erfindung sind somit auch transgene Pflanzenzellen, in denen die Aktivität eines erfindungsgemäßen Proteins verringert ist im Vergleich zu nicht-transformierten Pflanzen.
Die Herstellung von Pflanzenzellen mit einer verringerten Aktivität eines erfindungsgemäßen Proteins kann beispielsweise erzielt werden durch die Expression einer entsprechenden antisense-RNA, einer sense-RNA zur Erzielung eines Cosuppressionseffektes oder die Expression eines entsprechend konstruierten Ribozyms, das spezifisch Transkripte spaltet, die eines der erfindungsgemäßen Proteine codieren, unter Verwendung der erfindungsgemäßen Nucleinsäuremoleküle.
Vorzugsweise wird zur Reduzierung der Aktivität eines erfindungsgemäßen Proteins in pflanzlichen Zellen eine antisense-RNA exprimiert.
Hierzu kann zum einen ein DNA-Molekül verwendet werden, das die gesamte für ein erfindungsgemäßes Protein codierende Sequenz einschließlich eventuell vorhandener flankierender Sequenzen umfaßt, als auch DNA-Moleküle, die nur Teile der codierenden Sequenz umfassen, wobei diese Teile lang genug sein müssen, um in den Zellen einen antisense-Effekt zu bewirken. Es können im allgemeinen Sequenzen bis zu einer Mindestlänge von 15 bp, vorzugsweise einer Länge von 100-500 bp, für eine effiziente antisense-Inhibition insbesondere Sequenzen mit einer Länge über 500 bp verwendet werden. In der Regel werden DNA-Moleküle verwendet, die kürzer als 5000 bp, vorzugsweise Sequenzen, die kürzer als 2500 bp sind. Bevorzugt werden DNA-Moleküle verwendet, die homolog in bezug auf die zu transformierende Pflanzenspezies sind.
Möglich ist auch die Verwendung von DHA-Sequenzen, die einen hohen Grad an Homologie zu den Sequenzen der erfindungsgemäßen DNA-Moleküle aufweisen, aber nicht vollkommen identisch sind. Die minimale Homologie sollte größer als ca. 65 % sein. Die Verwendung von Sequenzen mit Homologien zwischen 95 und 100 % ist zu bevorzugen.
Die erfindungsgemäßen Zellen lassen sich daher von natürlicherweise vorkommenden Zellen dadurch unterscheiden, daß sie ein heterologes rekombinantes DNA-Molekül enthalten, das eine antisense-RNA, ein Ribozym oder eine Cosuppressions-RNA codiert. Aufgrund der Expression dieses heterologen rekombinanten DNA-Moleküls kommt es zur Verringerung der Synthese eines erfindungsgemäßen Proteins in den Zellen und somit zur Verringerung der entsprechenden Aktivität.
"Heterologe" DNA bedeutet in diesem Zusammenhang, daß es sich bei der in die Zellen eingeführten DNA um eine DNA handelt, die in dieser Form natürlicherweise nicht in den Zellen vorkommt. Es kann sich dabei zum einen um DNA handeln, die natürlicherweise in diesen transformierten Zellen gar nicht vorkommt oder auch um DNA, die, selbst wenn sie natürlicherweise in diesen Zellen vorkommt, als exogene DNA in andere genetische Loci integriert ist und sich somit in einer anderen genetischen Umgebung befindet.

Die erfindungsgemäßen transgenen Pflanzenzellen können nach dem Fachmann bekannten Techniken zu ganzen Pflanzen regeneriert werden. Gegenstand der Erfindung sind somit auch Pflanzen, die die erfindungsgemäßen transgenen Pflanzenzellen enthalten. Bei diesen Pflanzen kann es sich prinzipiell um Pflanzen jeder beliebigen Pflanzenspezies handeln, d.h. sowohl monokotyle als auch dikotyle Pflanzen. Vorzugsweise handelt es sich um Nutzpflanzen, d.h. um Pflanzen, die vom Menschen kultiviert werden für die menschliche oder tierische Ernährung oder für technische Zwecke. Insbesondere sind es vorzugsweise stärkesynthetisierende bzw. stärkespeichernde Pflanzen, wie z.B. Getreidearten (Roggen, Gerste, Hafer, Weizen, etc.), Reis, Mais, Erbse, Maniok oder Kartoffel. Die Erfindung betrifft ebenfalls Vermehrungsmaterial der erfindungsgemäßen Pflanzen, wie z.B. Früchte, Samen, Knollen, Wurzelstöcke, Sämlinge, Stecklinge etc.

Die erfindungsgemäßen transgenen Pflanzenzellen und Pflanzen synthetisieren aufgrund der Verringerung der Aktivität eines der erfindungsgemäßen Proteine eine Stärke, die beispielsweise in ihren physikalisch-chemischen Eigenschaften, insbesondere dem Amylose/Amylopektin-Verhältnis, dem Verzweigungsgrad, der durchschnittlichen Kettenlänge, dem Phosphatgehalt, dem Verkleisterungsverhalten, der Stärkekorngröße und/oder der Stärkekomform im Vergleich zu in Wildtyp-Pflanzen synthetisierter Stärke verändert ist. Diese Stärke kann beispielsweise veränderte Viskositäten und/oder Gelbildungseigenschaften ihrer Kleister zeigen im Vergleich zu Stärke aus Wildtyp-Pflanzen.

Gegenstand der Erfindung ist somit auch die aus den vorgehend beschriebenen transgenen Pflanzenzellen und Pflanzen erhältliche Stärke.

Die erfindungsgemäßen Stärken können nach dem Fachmann bekannten Verfahren modifiziert werden und eignen sich in unmodifizierter oder modifizierter Form für verschiedene Verwendungen im Nahrungsmittel- oder Nicht-Nahrungsmittel-bereich.

Grundsätzlich läßt sich die Einsatzmöglichkeit der Stärke in zwei große Bereiche unterteilen. Der eine Bereich umfaßt die Hydrolyseprodukte der Stärke, hauptsächlich Glucose und

Glucanbausteine, die über enzymatische oder chemische Verfahren erhalten werden. Sie dienen als Ausgangsstoff für weitere chemische Modifikationen und Prozesse, wie Fermentation. Für eine Reduktion der Kosten kann hierbei die Einfachheit und kostengünstige Ausführung eines Hydrolyseverfahrens von Bedeutung sein. Gegenwärtig verläuft es im wesentlichen enzymatisch unter Verwendung von Amyloglucosidase. Vorstellbar wäre eine Kosteneinsparung durch einen geringeren Einsatz von Enzymen. Eine Strukturveränderung der Stärke, z.B. Oberflächenvergrößerung des Korns, leichtere Verdaulichkeit durch geringeren Verzweigungsgrad oder eine sterische Struktur, die die Zugänglichkeit für die eingesetzten Enzyme begrenzt, könnte dies bewirken.

Der andere Bereich, in dem die Stärke wegen ihrer polymeren Struktur als sogenannte native Stärke verwendet wird, gliedert sich in zwei weitere Einsatzgebiete:
1. Nahrungsmittelindustrie
   Stärke ist ein klassischer Zusatzstoff für viele Nahrungsmittel, bei denen sie im wesentlichen die Funktion des Bindens von wäßrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell- und Verkleisterungstemperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Taustabilität, die Verdaulichkeit sowie die Fähigkeit zur Komplexbildung mit z.B. anorganischen oder organischen Ionen.
2. Nicht-Nahrungmittelindustrie
   In diesem großen Bereich kann die Stärke als Hilfsstoff für unterschiedliche Herstellungsprozesse bzw. als Zusatzstoff in technischen Produkten eingesetzt. Bei der
   Verwendung der Stärke als Hilfsstoff ist hier insbesondere die Papier- und Pappeindustrie zu nennen. Die Stärke dient dabei in erster Linie zur Retardation (Zurückhaltung von Feststoffen), der Abbindung von Füllstoff- und Feinstoffteilchen, als Festigungsstoff und zur Entwässerung. Darüber hinaus werden die günstigen Eigenschaften der Stärke in bezug auf die Steifigkeit, die Härte, den Klang, den Griff, den Glanz, die Glätte, die Spaltfestigkeit sowie die Oberflächen ausgenutzt.
2.1 Papier- und Pappeindustrie
   Innerhalb des Papierherstellungsprozesses sind vier Anwendungsbereiche, nämlich Oberfläche, Strich, Masse und Sprühen, zu unterscheiden.
   Die Anforderungen an die Stärke in bezug auf die Oberflächenbehandlung sind im wesentlichen ein hoher Weißegrad, eine angepaßte Viskosität, eine hohe Viskositätsstabilität, eine gute Filmbildung sowie eine geringe Staubbildung. Bei der Verwendung im Strich spielt der Feststoffgehalt, eine angepaßte Viskosität, ein hohes Bindevermögen sowie eine hohe Pigmentaffinität eine wichtige Rolle. Als Zusatz zur Masse ist eine rasche, gleichmäßige, verlustfreie Verteilung, eine hohe mechanische Stabilität und eine vollständige Zurückhaltung im Papierfließ von Bedeutung. Beim Einsatz der Stärke im Sprühbereich sind ebenfalls ein angepaßter Feststoffgehalt, hohe Viskosität sowie ein hohes Bindevermögen von Bedeutung.
2.2 Klebstoffindustrie
   Ein großer Einsatzbereich der Stärken besteht in der Klebstoffindustrie, wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90 % der Klebstoffe auf Stärkebasis werden in den Bereichen Wellpappenherstellung, Herstellung von Papiersäcken, Beuteln und Tüten, Herstellung von Verbundmaterialien für Papier und Aluminium, Herstellung von Kartonagen und Wiederbefeuchtungsleim für Briefumschläge, Briefmarken usw. eingesetzt.
2.3 Textil- und Textilpflegemittelindustrie
   Ein großes Einsatzfeld für die Stärken als Hilfmittel und Zusatzstoff ist der Bereich Herstellung von Textilien und Textilpflegemitteln. Innerhalb der Textilindustrie sind die folgenden vier Einsatzbereiche zu unterscheiden: Der Einsatz der Stärke als Schlichtmittel, d.h. als Hilfstoff zur Glättung und Stärkung des Klettverhaltens zum Schutz gegen die beim Weben angreifenden Zugkräfte sowie zur Erhöhung der Abriebfestigkeit beim Weben, Stärke als Mittel zur Textilaufrüstung vor allem nach qualitätsverschlechternden Vorbehandlungen, wie Bleichen, Färben usw., Stärke als Verdickungsmittel bei der Herstellung von Farbpasten zur Verhinderung von Farbstoffdiffusionen sowie Stärke als Zusatz zu Kettungsmitteln für Nähgarne.
2.4 Baustoffindustrie
   Der vierte Einsatzbereich ist die Verwendung der Stärken als Zusatz bei Baustoffen. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet. Weitere Einsatzbereiche sind die Beimischung zu Putz- und Mineralfasern. Bei Transportbeton werden Stärkeprodukte zur Verzögerung der Abbindung eingesetzt.
2.5 Bodenstabilisation
   Ein weiterer Markt für die Stärke bietet sich bei der Herstellung von Mitteln zur Bodenstabilisation an, die bei künstlichen Erdbewegungen zum temporären Schutz der Bodenpartikel gegenüber Wasser eingesetzt werden. Kombinationsprodukte aus der Stärke und Polymeremulsionen sind nach heutiger Kenntnis in ihrer Erosions- und verkrustungsmindernden Wirkung den bisher eingesetzten Produkten gleichzusetzen, liegen preislich aber deutlich unter diesen.
2.6 Einsatz bei Pflanzenschutz- und Düngemitteln
   Ein Einsatzbereich liegt bei der Verwendung der Stärke in Pflanzenschutzmitteln zur Veränderung der spezifischen Eigenschaften der Präparate. So kann die Stärke zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt werden.
2.7 Pharmaka, Medizin und Kosmetikindustrie
   Ein weiteres Einsatzgebiet besteht im Bereich der Pharmaka, Medizin und Kosmetikindustrie. In der pharmazeutischen Industrie kann die Stärke als Bindemittel für Tabletten oder zur Bindemittelverdünnung in Kapseln eingesetzt werden. Weiterhin kann die Stärke als Tablettensprengmittel dienen, da sie nach dem Schlucken Flüssigkeit absorbieren und nach kurzer Zeit soweit quellen, daß der Wirkstoff freigesetzt wird. Medizinische Gleit- und Wundpuder basieren aus qualitativen Gründen auf Stärke. Im Bereich der Kosmetik werden Stärken beispielsweise als Träger von Puderzusatzstoffen, wie Düften und Salicylsäure eingesetzt. Ein relativ großer Anwendungsbereich für die Stärke liegt bei Zahnpasta.
2.8 Stärkezusatz zu Kohlen und Briketts
   Einen Einsatzbereich bietet die Stärke als Zusatzstoff zu Kohle und Brikett. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6 %, bei kalorierter Kohle zwischen 0,1 und 0,5 %. Des weiteren gewinnen Stärken als Bindemittel an Bedeutung, da durch ihren Zusatz zu Kohle und Brikett der Ausstoß schädlicher Stoffe deutlich vermindert werden kann.
2.9 Erz- und Kohleschlammaufbereitung
   Die Stärke kann ferner bei der Erz- und Kohleschlammaufbereitung als Flockungsmittel eingesetzt werden.
2.10 Gießereihilfsstoff
   Ein weiterer Einsatzbereich besteht als Zusatz zu Gießereihilfsstoffen. Bei verschiedenen Gußverfahren werden Kerne benötigt, die aus Bindemittel-versetzten Sänden hergestellt werden. Als Bindemittel wird heute überwiegend Bentonit eingesetzt, das mit modifizierten Stärken, meist Quellstärken, versetzt ist.
   Zweck des Stärkezusatzes ist die Erhöhung der Fließfestigkeit sowie die Verbesserung der Bindefestigkeit. Darüber hinaus können die Quellstärken weitere produktionstechnische Anforderungen, wie im kalten Wasser dispergierbar, rehydratisierbar, gut in Sand mischbar und hohes Wasserbindungsvermögen, aufweisen.
2.11 Einsatz in der Kautschukindustrie
   In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens, dafür wird Stärke vor der Kaltvulkanisation auf die klebrigen gummierten Flächen von Kautschukstoffen gestreut, sowie die Verbesserung der Bedruckbarkeit des Kautschuks.
2.12 Herstellung von Lederersatzstoffen
   Eine weitere Absatzmöglichkeit der modifizierten Stärken besteht bei der Herstellung von Lederersatzstoffen.
2.13 Stärke in synthetischen Polymeren
   Auf dem Kunststoffsektor zeichnen sich folgende Einsatzgebiete ab: die Einbindung von Stärkefolgeprodukten in den Verarbeitungsprozess (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).

Die Verwendung der Stärke als reinem Füllstoff ist verglichen mit den anderen Stoffen wie Talkum nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyäthylen. Hierbei werden die Stärke und das synthetische Polymer durch Koexpression im Verhältnis von 1 : 1 zu einem 'master batch' kombiniert, aus dem mit granuliertem Polyäthylen unter Anwendung herkömmlicher Verfahrenstechniken diverse Produkte hergestellt werden. Durch die Einbindung von Stärke in Polyäthylenfolien kann eine erhöhte Stoffdurchlässigkeit bei Hohlkörpern, eine verbesserte Wasserdampfdurchlässigkeit, ein verbessertes Antistatikverhalten, ein verbessertes Antiblockverhalten sowie eine verbesserte Bedruckbarkeit mit wäßrigen Farben erreicht werden.
Eine andere Möglichkeit ist die Anwendung der Stärke in Polyurethanschäumen. Mit der Adaption der Stärkederivate sowie durch die verfahrenstechnische Optimierung ist es möglich, die Reaktion zwischen synthetischen Polymeren und den Hydroxygruppen der Stärken gezielt zu steuern. Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurchlässigkeit ohne Veränderung der Wasseraufnahme, Verringerung der Entflammbarkeit und der Aufrißdichte, kein Abtropfen brennbarer Teile, Halogenfreiheit und verminderte Alterung. Nachteile, die gegenwärtig noch vorhanden sind, sind verringerte Druckfestigkeit sowie eine verringerte Schlagfestigkeit.
Die Produktentwicklung beschränkt sich inzwischen nicht mehr nur auf Folien. Auch feste Kunststoffprodukte, wie Töpfe, Platten und Schalen, sind mit einem Stärkegehalt von über 50 % herzustellen. Des weiteren sind Stärke/ Polymermischungen günstig zu beurteilen, da sie eine sehr viel höhere biologische Abbaubarkeit aufweisen.
Außerordentliche Bedeutung haben weiterhin auf Grund ihres extremen Wasserbindungsvermögen Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfpolymerisate zeichnen sich durch ein besseres Binde- und Rückhaltevermögen von bis zu 1000 g Wasser pro g Stärke bei hoher Viskosität aus. Die Anwendungsbereiche für diese Superabsorber haben sich in den letzten Jahren stark ausgeweitet und liegen im Hygienebereich mit Produkten Windeln und Unterlagen sowie im landwirtschaftlichen Sektor, z.B. bei Saatgutpillierungen.

Entscheidend für den Einsatz der neuen, gentechnisch veränderten Stärken sind zum einen die Struktur, Wassergehalt, Proteingehalt, Lipidgehalt, Fasergehalt, Asche/Phosphatgehalt, Amylose/Amylopektinverhältnis, Molmassenverteilung, Verzweigungsgrad, Korngröße und -form sowie Kristallinität, zum anderen auch die Eigenschaften, die in folgende Merkmale münden: Fließ- und Sorptionsverhalten, Verkleisterungstemperatur, Viskosität, Dickungsleistung, Löslichkeit, Kleisterstruktur und - transparenz, Hitze-, Scher- und Säurestabilität, Retrogradationsneigung, Gelbildung, Gefrier/Taustabilität, Komplexbildung, Jodbindung, Filmbildung, Klebekraft, Enzymstabilität, Verdaulichkeit und Reaktivität.
Die Erzeugung modifizierter Stärken mittels gentechnischer Eingriffe in einer transgenen Pflanze kann zum einen die Eigenschaften der aus der Pflanze gewonnenen Stärke dahingehend verändern, daß weitere Modifikationen mittels chemischer oder physikalischer Verfahren nicht mehr notwendig erscheinen. Zum anderen können die durch gentechnische Verfahren veränderte Stärken weiteren chemischen Modifikationen unterworfen werden, was zu weiteren Verbesserungen der Qualität für bestimmte der oben beschriebenen Einsatzgebiete führt. Diese chemischen Modifikationen sind grundsätzlich bekannt. Insbesondere handelt es sich dabei um Modifikationen durch
- Hitzebehandlung,
- Säurebehandlung,
- Oxidation und
- Veresterungen,
welche zur Entstehung von Phosphat-, Nitrat-, Sulfat-, Xanthat-, Acetat- und Citratstärken führen. Weitere organische Säuren können ebenfalls zur Veresterung eingesetzt werden:
- Erzeugung von Stärkeethern Stärke-Alkylether, O-Allylether, Hydroxylalkylether, O-Carboxylmethylether, N-haltige Stärkeether, P-haltige Stärkeether, S-haltige Stärkeether
- Erzeugung von vernetzten Stärken
- Erzeugung von Stärke-Pfropf-Polymerisaten

Zur Expression der erfindungsgemäßen Nucleinsäuremoleküle in sense- oder antisense-Orientierung in pflanzlichen Zellen werden diese mit regulatorischen DNA-Elementen verknüpft, die die Transkription in pflanzlichen Zellen gewährleisten. Hierzu zählen insbesondere Promotoren. Generell kommt für die Expression jeder in pflanzlichen Zellen aktive Promotor in Frage.
Der Promotor kann dabei so gewählt sein, daß die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein. Sinnvolle Promotoren sind z.B. der Promotor der 35S RNA des Cauliflower Mosaic Virus und der Ubiquitin-Promotor aus Mais für eine konstitutive Expression, der Patatingen-Promotor B33 (Rocha-Sosa et al., EMBO J. 8 (1989), 23-29) für eine knollenspezifische Expression in Kartoffeln oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451) oder für eine endosperm-spezifische Expression der HMG-Promotor aus Weizen, der USP-Promotor, der Phaseolinpromotor oder Promotoren von Zein-Genen aus Mais.
Ferner kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. Gielen et al., EMBO J. 8 (1989), 23-29) und sind beliebig austauschbar.
Die vorliegende Erfindung stellt Nucleinsäuremoleküle zur Verfügung, die eine neue in Mais identifizierte Isoform einer Stärkesynthase codierenden. Dies erlaubt nun sowohl die Identifizierung der Funktion dieser Isoform bei der Stärkebiosynthese, als auch die Herstellung gentechnisch veränderter Pflanzen, bei denen die Aktivität dieses Enzyms verändert ist. Dies ermöglicht die Synthese einer Stärke mit veränderter Struktur und somit veränderten physikalisch-chemischen Eigenschaften in derartig manipulierten Pflanzen.
Die erfindungsgemäßen Nucleinsäuremoleküle können prinzipiell auch dazu verwendet werden, Pflanzen herzustellen, bei denen die Aktivität der erfindungsgemäßen Stärkesynthase erhöht oder verringert ist und gleichzeitig die Aktivitäten anderer, an der Stärkebiosynthese beteiligter Enzyme verändert sind. Dabei sind alle Kombinationen und Permutationen denkbar. Durch die Veränderung der Aktivitäten einer oder mehrerer Isoformen der Stärkesynthasen in Pflanzen kommt es zur Synthese einer in ihrer Struktur veränderten Stärke. Durch die Steigerung der Aktivität einer oder mehrerer Isoformen der Stärkesynthasen in den Zellen der stärkespeichernden Gewebe transformierter Pflanzen wie z.B. in dem Endosperm von Mais oder Weizen oder in der Knolle bei der Kartoffel kann es darüber hinaus zu einer Ertragssteigerung kommen. Beispielsweise können Nucleinsäuremoleküle, die für ein erfindungsgemäßes Protein codieren oder entsprechende antisense-Konstrukte, in Pflanzenzellen eingebracht werden, bei denen bereits die Synthese endogener GBSS I-, SSS- oder GBSS II-Proteine aufgrund eines antisense-Effektes oder einer Mutation inhibiert ist oder die Synthese des Verzweigungsenzyms inhibiert ist (wie z.B. beschrieben in WO92/14827 oder der ae-Mutante (Shannon und Garwood, 1984, in Whistler, BeMiller und Paschall, Starch:Chemistry and Technology, Academic Press, London, 2nd Edition: 25-86)).
Soll die Inhibierung der Synthese mehrerer Stärke-Synthasen in transformierten Pflanzen erreicht werden, so können DNA-Moleküle zur Transformation verwendet werden, die gleichzeitig mehrere, die entsprechenden Stärkesynthasen codierenden Regionen in antisense-Orientierung unter der Kontrolle eines geeigneten Promotors enthalten. Hierbei kann alternativ jede Sequenz unter der Kontrolle eines eigenen Promotors stehen, oder die Sequenzen können als Fusion von einem gemeinsamen Promotor transkribiert werden. Letztere Alternative wird in der Regel vorzuziehen sein, da in diesem Fall die Synthese der entsprechenden Proteine in etwa gleichem Maße inhibiert werden sollte.
Weiterhin ist die Konstruktion von DNA-Molekülen möglich, bei denen neben DNA-Sequenzen, die Stärkesynthasen codieren, weitere DNA-Sequenzen, die andere Proteine, die an der Stärkesynthese oder -modifikation beteiligt sind, in antisense-Orientierung an einen geeigneten Promotor gekoppelt sind. Die Sequenzen können hierbei wiederum hintereinandergeschaltet sein und von einem gemeinsamen Promotor transkribiert werden. Für die Länge der einzelnen codierenden Regionen, die in einem derartigen Konstrukt verwendet werden, gilt das, was oben bereits für die Herstellung von antisense-Konstrukten ausgeführt wurde. Eine obere Grenze für die Anzahl der in einem derartigen DNA-Molekül von einem Promotor aus transkribierten antisense-Fragmente gibt es nicht. Das entstehende Transkript sollte aber in der Regel eine Länge von 10 kb, vorzugsweise von 5 kb nicht überschreiten.
Codierende Regionen, die in derartigen DNA-Molekülen in Kombination mit anderen codierenden Regionen in antisense-Orientierung hinter einem geeigneten Promotor lokalisiert sind, können aus DNA-Sequenzen stammen, die für folgende Proteine codieren: Stärkekorn-gebundene (GBSS I und II) und lösliche Stärkesynthasen (SSS I und II), Verzweigungsenzyme, "Debranching"-Enzyme, Disproportionierungsenzyme und Stärkephosphorylasen. Dies ist nur eine beispielhafte Aufzählung. Auch die Verwendung anderer DNA-Sequenzen im Rahmen einer derartigen Kombination ist denkbar.
Mit Hilfe derartiger Konstrukte ist es möglich, in Pflanzenzellen, die mit diesen transformiert wurden, die Synthese mehrerer Enzyme gleichzeitig zu inhibieren.
Weiterhin können die Konstrukte in klassische Mutanten eingebracht werden, die für ein oder mehrere Gene der Stärkebiosynthese defekt sind (Shannon und Garwood, 1984, in Whistler, BeMiller und Paschall, Starch:Chemistry and Technology, Academic Press, London, 2nd Edition: 25-86). Diese Defekte können sich auf folgende Proteine beziehen: Stärkekorn-gebundene (GBSS I und II) und lösliche Stärkesynthasen (SSS I und II), Verzweigungsenzyme (BE I und II), "Debranching"-Enzyme (R-Enzyme), Disproportionierungsenzyme und Stärkephosphorylasen. Dies ist nur eine beispielhafte Aufzählung.
Mit Hilfe einer derartigen Vorgehensweise ist es weiterhin möglich, in Pflanzenzellen, die mit diesen transformiert wurden, die Synthese mehrerer Enzyme gleichzeitig zu inhibieren. Zur Vorbereitung der Einführung fremder Gene in höhere Pflanzen stehen eine große Anzahl von Clonierungsvektoren zur Verfügung, die ein Replikationssignal für E.coli und ein Markergen zur Selektion transformierter Bakterienzellen enthalten. Beispiele für derartige Vektoren sind pBR322, pUC-Serien, M13mp-Serien, pACYC184 usw.. Die gewünschte Sequenz kann an einer passenden Restriktionsschnittstelle in den Vektor eingeführt werden. Das erhaltene Plasmid wird für die Transformation von E.coli-Zellen verwendet. Transformierte E.coli-Zellen werden in einem geeigneten Medium gezüchtet, anschließend geerntet und lysiert. Das Plasmid wird wiedergewonnen. Als Analysemethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid DNA gespalten und gewonnene DNA-Fragmente mit anderen DNA-Sequenzen verknüpft werden. Jede Plasmid-DNA-Sequenz kann in den gleichen oder anderen Plasmiden cloniert werden.
Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von Agrobacterium tumefaciens oder Agrobacterium rhizogenes als Transformationsmittel, die Fusion von Protoplasten, die Injektion, die Elektroporation von DNA, die Einbringung von DNA mittels der biolistischen Methode sowie weitere Möglichkeiten.
Bei der Injektion und Elektroporation von DNA in Pflanzenzellen werden an sich keine speziellen Anforderungen an die verwendeten Plasmide gestellt. Es können einfache Plasmide wie z.B. pUC-Derivate verwendet werden. Sollen aber aus derartig transformierten Zellen ganze Pflanzen regeneriert werden, ist die Anwesenheit eines selektierbaren Markergens notwendig.
Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der Ti- und Ri-Plasmid T-DNA als Flankenbereich mit den einzuführenden Genen verbunden werden.
Werden für die Transformation Agrobakterien verwendet, muß die einzuführende DNA in spezielle Plasmide cloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E.coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al. Mol. Gen. Genet. 163 (1978), 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet.
Die Verwendung von T-DNA für die Transformation von Pflanzenzellen ist intensiv untersucht und ausreichend in EP 120 516; Hoekema, In: The Binary Plant Vector System Offsetdrukkerij Kanters B.V., Alblasserdam (1985), Chapter V; Fraley et al., Crit. Rev. Plant. Sci., 4, 1-46 und An et al. EMBO J. 4 (1985), 277-287 beschrieben worden.
Für den Transfer der DNA in die Pflanzenzelle können Pflanzen-Explantate zweckmäßigerweise mit Agrobacterium tumefaciens oder Agrobacterium rhizogenes kokultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplastentransformation sind bekannt (vgl. z.B. Willmitzer, L., 1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basel-Cambridge).
Während die Transformation dikotyler Pflanzen über Ti-Plasmid-Vektorsysteme mit Hilfe von Agrobacterium tumefaciens wohl etabliert ist, weisen neuere Arbeiten darauf hin, daß auch monokotyle Pflanzen der Transformation mittels Agrobacterium basierender Vektoren sehr wohl zugänglich sind (Chan et al., Plant Mol. Biol. 22 (1993), 491-506; Hiei et al., Plant J. 6 (1994), 271-282).
Alternative Systeme zur Transformation von monokotylen Pflanzen sind die Transformation mittels des biolistischen Ansatzes, die Protoplastentransformation, die Elektroporation von partiell permeabilisierten Zellen, die Einbringung von DNA mittels Glasfasern.

Spezifisch die Transformation von Mais wird in der Literatur verschiedentlich beschrieben (vgl. z.B. WO95/06128, EP 0 513 849; EP 0 465 875). In EP 292 435 wird ein Verfahren beschrieben, mit Hilfe dessen, ausgehend von einem schleimlosen, weichen (friable) granulösen Mais-Kallus, fertile Pflanzen erhalten werden können. Shillito et al. (Bio/Technology 7 (1989), 581) haben in diesem Zusammenhang beobachtet, daß es ferner für die Regenerierbarkeit zu fertilen Pflanzen notwendig ist, von Kallus-Suspensionskulturen auszugehen, aus denen eine sich teilende Protoplastenkultur, mit der Fähigkeit zu Pflanzen zu regenerieren, herstellbar ist. Nach einer in vitro Kultivierungszeit von 7 bis 8 Monaten erhalten Shillito et al. Pflanzen mit lebensfähigen Nachkommen, die jedoch Abnormalitäten in der Morphologie und der Reproduktivität aufweisen. Prioli und Söndahl (Bio/Technology 7 (1989), 589) beschreiben die Regeneration und die Gewinnung fertiler Pflanzen aus Mais-Protoplasten der Cateto Mais-Inzuchtlinie Cat 100-1. Die Autoren vermuten, daß die Protoplasten-Regeneration zu fertilen Pflanzen abhängig ist von einer Anzahl verschiedener Faktoren, wie z.B. von Genotyp, vom physiologischen Zustand der DonorZellen und von den Kultivierungsbedingungen.
Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin oder Phosphinotricin u.a. vermittelt. Der individuelle gewählte Marker sollte daher die Selektion transformierter Zellen gegenüber Zellen, denen die eingeführte DNA fehlt, gestatten.
Die transformierten Zellen wachsen innerhalb der Pflanze in der üblichen Weise (siehe auch McCormick et al., Plant Cell Reports 5 (1986), 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften. Von den Pflanzenzellen können Samen gewonnen werden.
Es sollten zwei oder mehrere Generationen angezogen werden, um sicherzustellen, daß das phänotypische Merkmal stabil beibehalten und vererbt wird. Auch sollten Samen geerntet werden, um sicherzustellen, daß der entsprechende Phänotyp oder andere. Eigenarten erhalten geblieben sind.

### Die Beispiele erläutern die Erfindung.

### Verwendete Abkürzungen

- bp: Basenpaar
- GBSS: granule bound starch synthase (Stärkekorn-gebundene Stär kesynthase)
- IPTG: Isopropyl β-D-Thiogalacto-Pyranosid
- SS: starch synthase ( Stärkesynthase)
- SSS: soluble starch synthase (lösliche Stärkesynthase)
In den Beispielen verwendete Medien und Lösungen:

| | |
|---|---|
| 20 x SSC | 175,3 g NaCl |
| | 88,2 g Natrium-Citrat |
| | ad 1000 ml mit ddH₂O |
| | pH 7,0 mit 10 N NaOH |
| YT | 8 g Bacto-Yeast extract |
| | 5 g Bacto-Tryptone |
| | 5 g NaCl |
| | ad 1000 ml mit ddH₂O |

### Protoplastenisolierungsmedium (100 ml)

| | |
|---|---|
| Cellulase Onozuka R S (Meiji Seika, Japan) | 800 mg |
| Pectolyase Y 23 | 40 mg |
| KNO₃ | 200 mg |
| KH₂PO₄ | 136 mg |
| K₂HPO₄ | 47 mg |
| CaCl₂ 2H₂O | 147 mg |
| MgSO₄ 7H₂O | 250 mg |
| Bovine serum albumin (BSA) | 20 mg |
| Glucose | 4000 mg |
| Fructose | 4000 mg |
| Saccharose | 1000 mg |
| pH | 5,8 |
| Omolarität | 660 mosm. |

### Protoplastenwaschlösung 1: wie Protoplastenisolierlösung, aber ohne Cellulase, Pectolyase und BSA

### Transformationspuffer

| | | |
|---|---|---|
| a) | Glucose | 0,5 M |
| | MES | 0,1 % |
| | MgCl₂ 6H₂O | 25 mM |
| | pH | 5,8 |
| | auf 600 mosm. einstellen | |
| b) | PEG 6000-Lösung | |
| | Glucose | 0,5 M |
| | MgCl₂ 6H₂O | 100 mM |
| | Hepes | 20 mM |
| | pH | 6,5 |

Dem obigen Puffer unter b) wird PEG 6000 kurz vor Gebrauch der Lösung zugesetzt (40 Gew.-% PEG). Die Lösung wird durch ein 0,45 µm Sterilfilter filtriert.

### W5 Lösung

| | |
|---|---|
| CaCl₂ | 125 mM |
| NaCl | 150 mM |
| KCl | 5 mM |
| Glucose | 50 mM |

### Protoplasten-Kulturmedium (Angaben in mg/l)

| | |
|---|---|
| KNO₃ | 3000 |
| (NH₄)₂SO₄ | 500 |
| MgSO₄ 7H₂O | 350 |
| KH₂PO₄ | 400 |
| CaCl₂ 2H₂O | 300 |

Fe-EDTA und Spurenelemente wie im Murashige-Skoog-Medium (Physiol. Plant, 15 (1962), 473).

| | |
|---|---|
| m-Inosit | 100 |
| Thiamin HCl | 1,0 |
| Nicotinsäureamid | 0,5 |
| Pyridoxin HCl | 0,5 |
| Glycin | 2,0 |
| Glucuronsäure | 750 |
| Galacturonsäure | 750 |
| Galactose | 500 |
| Maltose | 500 |
| Glucose | 36.000 |
| Fructose | 36.000 |
| Saccharose | 30.000 |
| Asparagin | 500 |
| Glutamin | 100 |
| Prolin | 300 |
| Caseinhydrolysat | 500 |
| 2, 4 Dichlorphenoxyessigsäure (2,4-D) | 0,5 |
| pH | 5,8 |
| Osmolarität | 600 mosm. |

In den Beispielen werden die folgenden Methoden verwendet:
1. Clonierungsverfahren
   Zur Clonierung in E.coli wurde der Vektor pBluescript II SK (Stratagene) verwendet.
2. Bakterienstämme
   Für den Bluescript-Vektor und für die pUSP-Konstrukte wurde der E.coli-Stamm DH5α (Bethesda Research Laboratories, Gaithersburgh, USA) verwendet. Für die in vivo excision wurde der E.coli-Stamm XL1-Blue verwendet.
3. Transformation von Mais
   (a) Herstellung von Protoplasten der Zellinie DSM 6009
      Protoplastenisolierung
      2 - 4 Tage, vorzugsweise 3 Tage nach dem letzten Mediumswechsel einer Protoplastensuspensionskultur wird das Flüssigmedium abgesaugt und die zurückbleibenden Zellen mit 50 ml Protoplastenwaschlösung 1 gespült und nochmals trockengesaugt. Zu jeweils 2 g der geernteten Zellmasse wird 10 ml Protoplastenisolierungsmedium gegeben. Die resuspendierten Zellen und Zellaggregate werden bei 27 ± 2° C unter leichtem Schütteln (30 bis 40 rpm) 4 bis 6 h im Dunkeln inkubiert.
      Protoplastenreinigung
      Sobald die Freisetzung von mindestens 1 Mio. Protoplasten/ml erfolgt ist (mikroskopische Beobachtung), wird die Suspension durch ein Edelstahl- und Nylonsieb von 200 bzw. 45 µm Maschenwerte gesiebt. Die Kombination eines 100 µm und eines 60 µm Siebs ermöglicht die Abtrennung der Zellaggregate genauso gut. Das protoplastenhaltige Filtrat wird mikroskopisch beurteilt. Üblicherweise enthält es 98 - 99 % Protoplasten. Der Rest sind unverdaute Einzelzellen. Protoplastenpräparationen mit diesem Reinheitsgrad werden ohne zusätzliche Gradientenzentrifugation für Transformationsexperimente verwendet. Durch Zentrifugation (100 UpM im Aufschwingrotor (100 x g, 3 min) werden die Protoplasten sedimentiert. Der Überstand wird verworfen und die Protoplasten in Waschlösung 1 resuspendiert. Die Zentrifugation wird wiederholt und die Protoplasten danach im Transformationspuffer resuspendiert.
   (b) Protoplastentransformation
      Die in Tranformationspuffer resuspendierten Protoplasten werden bei einem Titer von 0,5 - 1 x 10⁶ Protoplasten/ml in 10 ml Portionen in 50 ml Polyallomer-Röhrchen eingefüllt. Die zur Transformation verwendete DNA wird in Tris-EDTA (TE) Puffer gelöst. Pro ml Protoplastensuspension werden 20 µg Plasmid-DNA zugegeben. Als Vektor wird dabei ein Phosphinotricinresistenz vermittelndes Plasmid verwendet (vgl. z.B. EP 0 513 849). Nach der DNA-Zugabe wird die Protoplastensuspension vorsichtig geschüttelt, um die DNA homogen in der Lösung zu verteilen. Sofort danach wird tropfenweise 5 ml PEG-Lösung zugetropft.
      Durch vorsichtiges Schwenken der Röhrchen wird die PEG-Lösung homogen verteilt. Danach werden nochmals 5 ml PEG-Lösung zugegeben und das homogene Durchmischen wiederholt. Die Protoplasten verbleiben 20 min der PEG-Lösung bei ± 2° C. Danach werden die Protoplasten durch 3-minütiges Zentrifugieren (100 g; 1000 Upm) sedimentiert. Der Überstand wird verworfen. Die Protoplasten werden durch vorsichtiges Schütteln in 20 ml W5-Lösung gewaschen und danach erneut zentrifugiert. Danach werden sie in 20 ml Protoplastenkulturmedium resuspendiert, nochmals zentrifugiert und erneut in Kulturmedium resuspendiert. Der Titer wird auf 6 - 8 x 10⁵ Protoplasten/ml eingestellt und die Protoplasten in 3 ml Portionen in Petrischalen (ø 60 mm, Höhe 15 mm) kultiviert. Die mit Parafilm versiegelten Petrischalen werden bei 25 ± 2° C im Dunkeln aufgestellt.
(c) Protoplastenkultur
   Während der ersten 2 - 3 Wochen nach der Protoplastenisolierung und -transformation werden die Protoplasten ohne Zugabe von frischem Medium kultiviert. Sobald sich die aus den Protoplasten regenerierten Zellen zu Zellaggregaten mit mehr als 20 - 50 Zellen entwickelt haben, wird 1 ml frisches Protoplastenkulturmedium zugegeben, das als Osmoticum Saccharose (90 g/l) enthält.
(d) Selektion transformierter Maiszellen und Pflanzenregeneration
   3 - 10 Tage nach der Zugabe von frischem Medium können die aus Protoplasten entstandenen Zellaggregate auf Agar-Medien mit 100 mg/l L-Phosphinothricin plattiert werden. N6-Medium mit den Vitaminen des Protoplastenkulturmediums, 90 g/l Saccharose und 1,0 mg/l 2,4D ist ebenso geeignet wie ein analoges Medium beispielsweise mit den Makro- und Mikronährsalzen des MS-Mediums (Murashige und Skoog (1962), siehe oben).
   Auf dem Selektivmedium können die aus stabil transformierten Protoplasten hervorgegangenen Kalli ungehindert weiterwachsen. Nach 3 - 5 Wochen, vorzugsweise 4 Wochen können die transgenen Kalli auf frisches Selektionsmedium transferiert werden, welches ebenfalls 100 mg/l L-Phosphinothricin enthält, das aber kein Auxin mehr enthält. Innerhalb von 3 - 5 Wochen differenzieren ca. 50 % der transgenen Maiskalli, die das L-Phosphinothricinacetyltransferase-Gen in ihr Genom integriert haben, auf diesem Medium in Gegenwart von L-Phosphinothricin erste Pflanzen.
(e) Aufzucht transgener Regeneratpflanzen
   Das embryogene transformierte Maisgewebe wird auf hormonfreiem N6-Medium (Chu C.C. et al., Sci. Sin. 16 (1975), 659) in Gegenwart von 5x10⁻⁴ M L-Phosphinothricin kultiviert. Auf diesem Medium entwickeln sich Maisembryonen, die das Phsphinothricinacetyltransferase-Gen (PAT-Gen) hinreichend stark exprimieren, zu Pflanzen. Nicht transformierte Embryonen oder solche mit nur sehr schwacher PAT-Aktivität sterben ab. Sobald die Blätter der in vitro-Pflanzen eine Länge von 4 - 6 mm erreicht haben, können diese in Erde transferiert werden. Nach Abwaschen von Agarresten an den Wurzeln werden die Pflanzen in ein Gemisch von Lehm,. Sand, Vermiculit und Einheitserde im Verhältnis 3:1:1:1 gepflanzt und während der ersten 3 Tage nach dem Verpflanzen bei 90 - 100 % relativer Luftfeuchte an die Erdkultur adaptiert. Die Anzucht erfolgt in einer Klimakammer mit 14 h Lichtperiode ca. 25000 Lux in Pflanzenhöhe bei einer Tag/Nachttemperatur von 23 ± 1/17 ± 1° C. Die adaptierten Pflanzen werden bei einer Luftfeuchte von 65 ± 5 % kultiviert.
4. Radioaktive Markierung von DNA-Fragmenten
   Die radioaktive Markierung von DNA-Fragmenten wurde mit Hilfe eines DNA-Random Primer Labelling Kits der Firma Boehringer (Deutschland) nach den Angaben des Herstellers durchgeführt.

### Beispiel 1

Identifizierung, Isolierung und Charakterisierung einer cDNA, die eine neue Isoform einer Stärkesynthase aus Zea mays codiert

Zur Identifizierung einer cDNA, die eine neue Stärkesynthase aus Mais codiert, wurde die Strategie der funktionellen Expression in einer geeigneten E. coli-Mutante gewählt. E. coli synthetisiert auf bestimmten Nährmedien (Vollmedium mit 1% Glucose) als Speicherkohlenhydrat Glycogen, ein Polysaccharid, das dem Amylopektin in seiner Struktur ähnlich ist, jedoch stärker verzweigt ist (7-10% Verzweigungspunkte gegenüber 4-5%) und normalerweise ein höheres Molekulargewicht aufweist. Der höhere Verzweigungsgrad bedingt auch eine unterschiedliche Färbung mit Jod. Glycogen wird mit Jod bräunlich, Amylopectin lila und Amylose blau angefärbt.
In E. coli sind essentiell drei Gene für die Glycogensynthese verantwortlich, glgA, glgB und glgC, die Glycogensynthase, das Verzweigungsenzym und die ADP-glucosepyrophosphorylase codieren. Dieses System ist analog dem der Stärkebiosynthese in Pflanzen. Wird eine pflanzliche Stärkesynthase in Wildtyp E. coli-Zellen exprimiert, so ist der Einfluß des Enzyms auf die Eigenschaften des Glycogens mittels Jodfärbung nur sehr schwer oder gar nicht zu detektieren, da die von der Stärkesynthase produzierten Glucane von dem Verzweigungsenzym genauso verzweigt werden, wie die von der Glycogensynthase produzierten Glucane.
Deshalb wurde ein E.coli-Stamm erstellt, der ein einfaches Screening nach der funktionellen Expression einer Stärkesynthase mittels Jodfärbung erlaubt. Hierfür wurde die Mutante HfrG6MD2 (Schwartz, J. Bacteriol. 92 (1966), 1083-1089), in der sämtliche glg-Gene deletiert sind, transformiert mit dem Plasmid pACAC. Dieses Plasmid enthält ein DNA-Fragment, das die ADP-Glucose-Pyrophosphorylase (AGPase) aus E. coli codiert, unter der Kontrolle des lac Z-Promotors. Das Fragment war als ca. 1,7 kb großes DraI/HaeII-Fragment aus dem Vektor pEcA-15 (siehe B. Müller-Röber (1992), Dissertation, FU Berlin) isoliert worden und nach Glättung der Enden in einen mit HindIII linearisierten pACYC184-Vektor cloniert worden. Dieses Plasmid vermittelt die Expression einer mutierten, deregulierten ADP-Glucose-Pyrophosphorylase aus dem E. coli-Stamm LCB 618, der größere Mengen an Glycogen, aufgrund der Mutation dieses Enzyms, akkumuliert (Preise und Romeo in Advances in Microbial Physiology, Academic Press, London, Vol. 30, 183-238). Dies gewährleistet die Bereitstellung ausreichender Mengen von ADP-Glucose, dem Substrat der Stärkesynthasen, und soll bei gleichzeitiger funktioneller Expression von Stärkesynthasen die Synthese von mit Jod blau anfärbbaren linearen α-1,4-Glucanen in E. coli HfrG6MD2 gewährleisten. Weiterhin ist das Plasmid pACAC als Derivat des Vektors pACYC184 mit Plasmiden wie pBluescript SK (-) kompatibel.
Dieser Stamm wurde mit einer cDNA-Bibliothek transformiert, die in dem Vektor pBluescript SK (-) enthalten war und aus RNA aus Blättern von Zea mays, Linie B73, hergestellt worden war. Diese wurde erstellt indem ca. 10⁶ Phagen einer cDNA-Bibliothek aus RNA aus Blättern von Zea mays, Linie B73, enthalten in dem Vektor Uni-ZAPTMXR (Stratagene GmbH,Heidelberg), mittels in vivo excision zunächst in Phagmide überführt wurden. E. coli XL1-Blue Zellen wurden mit diesen Phagmiden infiziert und 3x10⁵ Transformanden wurden auf festem, selektivem (enthaltend Ampicillin) Nährmedium plattiert. Nach Wachstum wurden die Zellen abgeschwemmt und aus diesen Plasmid-DNA präpariert. Der Transfer der Plasmid-DNA in die Bakterienzellen erfolgte nach der Methode von Hanahan (J. Mol. Biol. 166 (1983), 557-580). Ca. 4x10⁵ transformierte E. coli-Zellen wurden auf Agarkulturmedien mit folgender Zusammensetzung ausgestrichen:
YT-Medium mit:
1,5% Bacto-Agar
1 % Glucose
10 mg/l Chloramphenicol
50 mg/l Ampicillin
1 mM IPTG
2 mM Diaminopimelinsäure

Nach Inkubation über Nacht bei 37°C wurden die Zellen mit Jod bedampft. Eine blaufärbende Kolonie wurde erhalten. Aus dieser wurde Plasmid-DNA isoliert und diese zur nochmaligen Transformation eingesetzt. Die erhaltenen Transformanden wurden auf Replikaplatten ausgestrichen. Eine der Replikaplatten wurde wiederum angefärbt. Blaufärbende Clone wurden zur Präparation größerer Mengen an Plasmid-DNA angezogen.
Nach Überprüfung der Größe des cDNA-Fragments wurde der Clon pSSZm weiter analysiert.

### Beispiel 2

### Sequenzanalyse der cDNA-Insertion des Plasmids pSSZm

Aus einem entsprechend Beispiel 1 erhaltenen E. coli-Clon wurde das Plasmid pSSZm isoliert und seine cDNA-Insertion durch Standardverfahren mittels der Didesoxynucleotidmethode (Sanger et al., Proc. Natl. Acad. Sci. USA 74 (1977), 5463-5467) bestimmt. Die Insertion ist 2651 bp lang und stellt eine partielle cDNA dar. Die Nucleotidsequenz ist unter Seq ID No. 1 angegeben. Die korrespondierende Aminosäuresequenz ist unter Seq ID No. 2 dargestellt.
Eine Sequenzanalyse und ein Sequenzvergleich mit bekannten Sequenzen zeigte, daß die unter Seq ID No. 1 dargestellte Sequenz neu ist und eine partielle codierende Region umfaßt, die gewisse Homologien zu Stärkesynthasen aus verschiedenen Organismen aufweist. Weiterhin stellt das codierte Protein eine neue Isoform von Stärkesynthasen dar, daß sich den bisher beschriebenen Klassen nicht eindeutig zuordnen läßt. Das durch diese cDNA-Insertion oder durch hybridisierende Sequenzen codierte Protein wird im Rahmen dieser Anmeldung als SSZm bezeichnet. Mit Hilfe dieser partiellen cDNA-Sequenz ist es für eine in der Molekularbiologie erfahrene Person ohne weiteres möglich, die gesamte codierende Region enthaltende Vollängenclone zu isolieren und seine Sequenz zu bestimmen. Dazu wird z.B. eine blattspezifische cDNA-Expressionsbank aus Zea mays, Linie B73 (Stratagene GmbH, Heidelberg), nach Standardverfahren mittels Hybridisierung mit einem 5'-Fragment der cDNA-Insertion des Plasmids pSSZM (200 bp) auf Vollängen-Clone hin durchgemustert. So erhaltene Clone werden sodann sequenziert. Eine andere Möglichkeit zum Erhalt der noch fehlenden 5'-terminal gelegenen Sequenzen besteht in der Anwendung der 5'-Race Methode (Stratagene o.vgl. Hersteller).

## Patentansprüche

1. Nucleinsäuremolekül codierend ein Protein mit der biologischen Aktivität einer Stärkesynthase ausgewählt aus der Gruppe bestehend aus
(a) Nucleinsäuremolekülen, die ein Protein codieren, das die unter SEQ ID NO:2 angegebene Aminosäuresequenz umfaßt;
(b) Nucleinsäuremolekülen, die die unter SEQ ID NO:1 dargestellte Nucleotidsequenz umfassen oder eine korrespondierende Ribonucleotidsequenz;
(c) Nucleinsäuremolekülen, die mit den unter (a) oder (b) genannten Nucleinsäuremolekülen unter stringenten Bedingungen hybridisieren und ein Protein mit Stärkesynthase-Aktivität codieren; und
(d) Nucleinsäuremolekülen, deren codierender Bereich eine Sequenzidentität von mindestens 60% zu dem in SEQ ID NO:1 dargestellten codierenden Bereich aufweist und die ein Protein mit Stärkesynthase-Aktivität codieren;
sowie der komplementäre Strang eines solchen Nucleinsäuremoleküls.

2. Nucleinsäuremolekül nach Anspruch 1, das ein DNA-Molekül ist.

3. DNA-Molekül nach Anspruch 2, das ein cDNA-Molekül ist.

4. Nucleinsäuremolekül nach Anspruch 1, das ein RNA-Molekül ist.

5. Oligonucleotid, das spezifisch mit einem Nucleinsäuremolekül nach einem der Ansprüche 1 bis 4 hybridisiert.

6. Vektor enthaltend ein DNA-Molekül nach einem der Ansprüche 1 bis 4.

7. Vektor nach Anspruch 6, wobei das DNA-Molekül in sense-Orientierung mit regulatorischen Elementen verknüpft ist, die die Transkription und Synthese einer translatierbaren RNA in pro- oder eukaryontischen Zellen gewährleisten.

8. Wirtszellen, die mit einem Nucleinsäuremolekül nach einem der Ansprüche 1 bis 4 oder einem Vektor nach Anspruch 6 oder 7 transformiert und genetisch modifiziert ist.

9. Protein codiert durch ein Nucleinsäuremolekül nach einem der Ansprüche 1 bis 4.

10. Verfahren zur Herstellung eines Proteins nach Anspruch 9, bei dem eine Wirtszelle nach Anspruch 8 unter Bedingungen kultiviert wird, die die Synthese des Proteins erlauben, und das Protein aus den kultivierten Zellen und/oder dem Kulturmedium isoliert wird.

11. Transgene Pflanzenzelle, die mit einem Nucleinsäuremolekül nach einem der Ansprüche 1 bis 4 oder einem Vektor nach Anspruch 6 oder 7 transformiert wurde oder die von einer solchen Zelle abstammt, wobei das Nucleinsäuremolekül, das das Protein mit der biologischen Aktivität einer Stärkesynthase codiert, unter der Kontrolle regulatorischer Elemente steht, die die Transkription einer translatierbaren mRNA in pflanzlichen Zellen erlauben.

12. Pflanze enthaltend Pflanzenzellen nach Anspruch 11.

13. Pflanze nach Anspruch 12, die eine Nutzpflanze ist.

14. Pflanze nach Anspruch 13, die eine stärkespeichernde Pflanze ist.

15. Pflanze nach Anspruch 14, die eine Maispflanze ist.

16. Vermehrungsmaterial einer Pflanze nach einem der Ansprüche 12 bis 15 enthaltend Pflanzenzellen nach Anspruch 11.

17. Transgene Pflanzenzelle, **dadurch gekennzeichnet, daß** in dieser Pflanzenzelle die Aktivität eines Proteins nach Anspruch 9 verringert ist aufgrund der Expression eines heterologen rekombinanten DNA-Moleküls, das
(a) eine antisense-RNA codiert zu Transkripten von endogen in der Zelle vorliegenden Genen, die ein Protein nach Anspruch 9 codieren; und/oder
(b) ein Ribozym codiert, das spezifisch Transkripte spalted von endogen in der Zelle vorliegenden Genen, die ein Protein nach Anspruch 9 codieren; und/oder
(c) eine RNA codiert, die aufgrund eines Cosuppressionseffekts in den Zellen die Synthese eines Proteins nach Anspruch 9 inhibiert.

18. Pflanzenzelle nach Anspruch 17, wobei die Reduktion der Aktivität in dieser Zelle durch die Expression einer antisense-RNA zu Transkripten eines DNA-Moleküls nach Anspruch 1 erreicht wird.

19. Pflanze enthaltend Pflanzenzellen nach Anspruch 17 oder 18.

20. Pflanze nach Anspruch 19, die eine Nutzpflanze ist.

21. Pflanze nach Anspruch 20, die eine stärkespeichernde Pflanze ist.

22. Pflanze nach Anspruch 21, die eine Maispflanze ist.

23. Vermehrungsmaterial einer Pflanze nach einem der Ansprüche 19 bis 22, enthaltend Zellen nach Anspruch 17 oder 18.

## Claims

1. A nucleic acid molecule encoding a protein with the biological activity of a starch synthase selected from the group consisting of:
(a) nucleic acid molecules encoding a protein comprising the amino acid sequence indicated in SEQ ID NO: 2;
(b) nucleic acid molecules comprising the nucleotide sequence depicted in SEQ ID NO: 1 or a corresponding ribonucleotide sequence;
(c) nucleic acid molecules which hybridize under stringent conditions to the nucleic acid molecules indicated in (a) or (b) and which encode a protein with starch synthase activity; and
(d) nucleic acid molecules the coding region of which has a sequence identity of at least 60% to the coding region depicted in SEQ ID NO: 1 and which encode a protein with starch synthase activity;
or the complementary strand of such a nucleic acid molecule.

2. The nucleic acid molecule of claim 1 which is a DNA molecule.

3. The DNA molecule of claim 2 which is a cDNA molecule.

4. The nucleic acid molecule of claim 1 which is an RNA molecule.

5. An oligonucleotide which specifically hybridizes with a nucleic acid molecule of any one of claims 1 to 4.

6. A vector comprising a DNA molecule of any one of claims 1 to 4.

7. The vector of claim 6, wherein the DNA molecule is linked in sense-orientation to regulatory elements which ensure the transcription and the synthesis of a translatable RNA in prokaryotic or eukaryotic cells.

8. Host cells which are transformed and genetically modified with a nucleic acid molecule of any one of claims 1 to 4 or with a vector of claim 6 or 7.

9. A protein encoded by a nucleic acid molecule of any one of claims 1 to 4.

10. A method for the production of a protein of claim 9 in which a host cell of claim 8 is cultivated under conditions that allow for the synthesis of the protein and in which the protein is isolated from the cultivated cells and/or the culture medium.

11. A transgenic plant cell transformed with a nucleic acid molecule of any one of claims 1 to 4 or with a vector of claim 6 or 7, or derived from such a cell, wherein the nucleic acid molecule encoding the protein with the biological activity of a starch synthase is subject to the control of regulatory elements allowing for the transcription of a translatable mRNA in plant cells.

12. A plant comprising plant cells of claim 11.

13. The plant of claim 12 which is a useful plant.

14. The plant of claim 13 which is a starch-storing plant.

15. The plant of claim 14 which is a maize plant.

16. Propagation material of a plant of any one of claims 12 to 15, containing plant cells of claim 11.

17. A transgenic plant cell which is **characterized in that** the activity of a protein of claim 9 is reduced in this plant cell due to the expression of a heterologous recombinant DNA molecule which
(a) encodes an antisense-RNA to transcripts of genes which encode a protein of claim 9 and which occur endogenously in the cell; and/or
(b) encodes a ribozyme which specifically cleaves transcripts of genes which encode a protein of claim 9 and which occur endogenously in the cell; and/or
(c) encodes an RNA which inhibits the synthesis of a protein of claim 9 due to a cosuppression effect within the cells.

18. The plant cell of claim 17, wherein the reduction of the activity in said cell is achieved by the expression of an antisense-RNA to transcripts of a DNA molecule of claim 1.

19. A plant containing plant cells of claim 17 or 18.

20. The plant of claim 19 which is a useful plant.

21. The plant of claim 20 which is a starch-storing plant.

22. The plant of claim 21 which is a maize plant.

23. Propagation material of a plant of any one of claims 19 to 22 containing cells of claim 17 or 18.

## Revendications

1. Molécule d'acide nucléique codant pour une protéine avec l'activité biologique d'une amidon synthase choisie dans le groupe comprenant
(a) molécules d'acide nucléique qui codent pour une protéine qui comprend la séquence en acides aminés indiquée sous SEQ ID NO:2 ;
(b) molécules d'acide nucléique qui comprennent la séquence nucléotidique présentée sous SEQ ID NO:1 ou une séquence ribonucléotidique correspondante ;
(c) molécules d'acide nucléique qui s'hybrident avec les molécules d'acide nucléique nommées en (a) ou (b) sous des conditions stringentes et codent pour une protéine avec une activité amidon synthase ; et
(d) molécules d'acide nucléique dont le domaine codant montre une identité de séquence d'au moins 60 % au domaine codant présenté par SEQ ID NO:1 et qui codent pour une protéine avec une activité amidon synthase ;
ainsi que le brin complémentaire d'une telle molécule d'acide nucléique.

2. Molécule d'acide nucléique selon la revendication 1, qui est une molécule d'ADN.

3. Molécule d'ADN selon la revendication 2, qui est une molécule d'ADNc.

4. Molécule d'acide nucléique selon la revendication 1, qui est une molécule d'ARN.

5. Oligonucléotide qui s'hybride spécifiquement avec une molécule d'acide nucléique selon l'une des revendications 1 à 4.

6. Vecteur contenant une molécule d'ADN selon l'une des revendications 1 à 4.

7. Vecteur selon la revendication 6, où la molécule d'ADN est liée dans l'orientation sens avec des éléments de régulation qui garantissent la transcription et la synthèse d'un ARN traduisible dans des cellules pro- ou eucaryotes.

8. Cellule hôte qui est transformée avec par une molécule d'acide nucléique selon l'une des revendications 1 à 4 ou un vecteur selon la revendication 6 ou 7 et génétiquement modifiée.

9. Protéine codée par une molécule d'acide nucléique selon l'une des revendications 1 à 4.

10. Procédé pour la préparation d'une protéine selon la revendication 9, par lequel une cellule hôte selon la revendication 8 est cultivée sous des conditions qui permettent la synthèse protéique et la protéine est isolée des cellules cultivées et/ou du milieu de culture.

11. Cellule de plante transgénique qui a été transformée avec par une molécule d'acide nucléique selon l'une des revendications 1 à 4 ou un vecteur selon la revendication 6 ou 7 ou qui descend d'une telle cellule, où la molécule d'acide nucléique, qui code pour la protéine avec l'activité biologique d'une amidon synthase, se trouve sous le contrôle d'éléments de régulation qui permettent la transcription d'un ARNm traduisible dans des cellules de plantes.

12. Plante contenant des cellules de plante selon la revendication 11.

13. Plante selon la revendication 12, qui est une planté utile.

14. Plante selon la revendication 13, qui est une plante stockant de l'amidon.

15. Plante selon la revendication 14, qui est une plante de mais.

16. Matière de multiplication d'une plante selon l'une des revendications 12 à 15 contenant des cellules de plantes selon la revendication 11.

17. Cellule de plante transgénique **caractérisée en ce que**, dans cette cellule de plante, l'activité d'une protéine selon la revendication 9 est diminuée en raison de l'expression d'une molécule d'ADN hétérologue recombinante qui
(a) code pour un ARN antisens pour des transcrits de gènes endogènes présents dans la cellule qui codent pour une protéine selon la revendication 9 ; et/ou
(b) code pour un ribozyme qui décompose spécifiquement des transcrits de gènes endogènes présents dans la cellule qui codent pour une protéine selon la revendication 9 ; et/ou
(c) code pour un ARN qui inhibe la synthèse protéique selon la revendication 9 en raison d'un effet co-suppresseur dans les cellules.

18. Cellule de plante selon la revendication 17, où la réduction de l'activité dans cette cellule est atteinte par l'expression d'un ARN antisens pour des transcrits d'une molécule d'ADN selon la revendication 1.

19. Plante contenant des cellules de plantes selon la revendication 17 ou 18.

20. Plante selon la revendication 19, qui est une plante utile.

21. Plante selon la revendication 20, qui est une plante stockant de l'amidon.

22. Plante selon la revendication 21, qui est une plante de mais.

23. Matière de multiplication d'une plante selon l'une des revendications 19 à 22 contenant des cellules de plantes selon la revendication 17 ou 18.


### SEQUENZPROTOKOLL

(1) ALLGEMEINE ANGABEN:
   (i) ANMELDER:
      (A) NAME: Jens Koßmann
      (B) STRASSE: Golmer Fichten 9
      (C) ORT: Golm
      (E) LAND: Deutschland
      (F) POSTLEITZAHL: 14476
   (ii) BEZEICHNUNG DER ERFINDUNG: Nucleinsaeuremolekuele aus Pflanzen codierend Enzyme, die an der Staerkesynthese beteiligt sind
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 2652 Basenpaare
      (B) ART: Nucleotid
      (C) STRANGFORM: Einzelstrang
      (D) TOPOLOGIE: linear
   (ii) ART DES MOLEKÜLS: cDNA zu mRNA
   (iii) HYPOTHETISCH: NEIN
   (iv) ANTISENSE: NEIN
   (vi) URSPRÜNGLICHE HERKUNFT:
      (A) ORGANISMUS: Zea mays
      (B) STAMM: B73
      (F) GEWEBETYP: Blattgewebe
   (vii) UNMITTELBARE HERKUNFT:
      (A) BIBLIOTHEK: cDNA-Bibliothek in pBluescriptSK-
      (B) CLON(E): pSSZm
   (ix) MERKMAL:
      (A) NAME/SCHLÜSSEL: CDS
      (B) LAGE:9..2213
   (xi) SEQUENZBESCHREIBUNG: SEQ ID NO: 1:
   GAATTCGG CAC GAG AAT TTT CTA AAA GGA AAG CTT ATT GAG ATA ACT GAG        50
            His Glu Asn Phe Leu Lys Gly Lys Leu Ile Glu Ile Thr Glu
              1               5                  10
   ACA GAG GAG AGT CTA TTC AAG TTG GAG AAA GAG TGT GCT CTT CTA AAT         98
   Thr Glu Glu Ser Leu Phe Lys Leu Glu Lys Glu Cys Ala Leu Leu Asn
    15                  20                  25                  30
   GCT TCC CTT AGG GAG CTC GAG TGT ACA TCC ACT TCT GCC CAA TCT GAT        146
   Ala Ser Leu Arg Glu Leu Glu Cys Thr Ser Thr Ser Ala Gln Ser Asp
                    35                  40                  45
   GTG TTG AAA CTT GGC CCT CTG CAA CAA GAT GCC TGG TGG GAG AAA GTA        194
   Val Leu Lys Leu Gly Pro Leu Gln Gln Asp Ala Trp Trp Glu Lys Val
                50                  55                  60
   GAA AAT TTG GAA GAC TTG CTT GAT TCC ACA GCA AAC CAA GTG GAG CAT        242
   Glu Asn Leu Glu Asp Leu Leu Asp Ser Thr Ala Asn Gln Val Glu His
            65                  70                  75
   GCT TCT TTG ACG CTA GAT GGT TAC CGT GAT TTC CAG GAT AAG GTT GAC        290
   Ala Ser Leu Thr Leu Asp Gly Tyr Arg Asp Phe Gln Asp Lys Val Asp
        80                  85                  90
   AAA CTA AAA GCA TCA TTG GGA ACA ACA AAC GTA TCA GAG TTC TGT CTT        338
   Lys Leu Lys Ala Ser Leu Gly Thr Thr Asn Val Ser Glu Phe Cys Leu
    95                 100                 105                 110
   TAT TTG GTT GAT ATT TTG CAG CAA AGG GTA AAA TCA GTA GAA GAG CGC        386
   Tyr Leu Val Asp Ile Leu Gln Gln Arg Val Lys Ser Val Glu Glu Arg
                   115                 120                 125
   TTT CAA GCA TGT AAT CAT GAA ATG CAT TCA CAA ATT GAA CTT TAT GAA        434
   Phe Gln Ala Cys Asn His Glu Met His Ser Gln Ile Glu Leu Tyr Glu
               130                 135                 140
   CAC TCA ATA GTG GAG TTT CAT GGT ACT CTC AGC AAA CTA ATA AAT GAA        482
   His Ser Ile Val Glu Phe His Gly Thr Leu Ser Lys Leu Ile Asn Glu
           145                 150                 155
   AGT GAG AAA AAG TCA ATG GAG CAT TAT GCA GAA GGC ATG CCA TCA GAG        530
   Ser Glu Lys Lys Ser Met Glu His Tyr Ala Glu Gly Met Pro Ser Glu
       160                 165                 170
   TTC TGG AGT AGG ATC TCT CTT CTG ATT GAT GGG TGG TCG CTT GAG AAG        578
   Phe Trp Ser Arg Ile Ser Leu Leu Ile Asp Gly Trp Ser Leu Glu Lys
   175                 180                 185                 190
   AAA ATA TCC ATT AAT GAT GCA AGT ATG TTG AGA GAA ATG GCT TGG AAA        626
   Lys Ile Ser Ile Asn Asp Ala Ser Met Leu Arg Glu Met Ala Trp Lys
                   195                 200                 205
   AGG GAT AAT CGC CTC CGG GAA GCT TAC TTG TCA TCC AGA GGA ATG GAA        674
   Arg Asp Asn Arg Leu Arg Glu Ala Tyr Leu Ser Ser Arg Gly Met Glu
               210                 215                 220
   GAG AGG GAA CTG ATA GAT AGT TTT CTA AAG ATG GCA CTA CCA GGA ACA     722
   Glu Arg Glu Leu Ile Asp Ser Phe Leu Lys Met Ala Leu Pro Gly Thr
           225                 230                 235
   AGT TCT GGT TTG CAC ATT GTC CAC ATA GCA GCA GAG ATG GCT CCT GTC     770
   Ser Ser Gly Leu His Ile Val His Ile Ala Ala Glu Met Ala Pro Val
       240                 245                 250
   GCA AAG GTT GGT GGT CTG GCA GAT GTG ATC TCT GGT CTT GGG AAG GCA     818
   Ala Lys Val Gly Gly Leu Ala Asp Val Ile Ser Gly Leu Gly Lys Ala
   255                 260                 265                 270
   CTT CAA AAA AAG GGG CAC CTT GTA GAG ATT ATT CTT CCC AAA TAT GAT     866
   Leu Gln Lys Lys Gly His Leu Val Glu Ile Ile Leu Pro Lys Tyr Asp
                   275                 280                 285
   TGC ATG CAG CAT AAC CAA ATA AAT AAT CTT AAG GTT CTA GAT GTT GTG     914
   Cys Met Gln His Asn Gln Ile Asn Asn Leu Lys Val Leu Asp Val Val
               290                 295                 300
   GTG AAG TCT TAC TTT GAA GGA AAT ATG TTT GCC AAC AAG ATA TGG ACT     962
   Val Lys Ser Tyr Phe Glu Gly Asn Met Phe Ala Asn Lys Ile Trp Thr
           305                 310                 315
   GGA ACT GTT GAA GGT CTT CCG GTC TAC TTT ATT GAA CCG CAA CAT CCA     1010
   Gly Thr Val Glu Gly Leu Pro Val Tyr Phe Ile Glu Pro Gln His Pro
       320                 325                 330
   GGT AAG TTC TTC TGG AGG GCA CAA TAC TAC GGA GAG CAT GAT GAC TTC     1058
   Gly Lys Phe Phe Trp Arg Ala Gln Tyr Tyr Gly Glu His Asp Asp Phe
   335                 340                 345                 350
   AAA CGT TTT TCG TAC TTT AGC CGT GTT GCA CTG GAA TTG CTT TAC CAA     1106
   Lys Arg Phe Ser Tyr Phe Ser Arg Val Ala Leu Glu Leu Leu Tyr Gln
                   355                 360                 365
   TCT GGG AAG AAA GTT GAC ATA ATT CAC TGC CAT GAC TGG CAG ACT GCA     1154
   Ser Gly Lys Lys Val Asp Ile Ile His Cys His Asp Trp Gln Thr Ala
               370                 375                 380
   TTT GTT GCA CCT CTT TAC TGG GAT GTA TAT GCA AAC CTG GGC TTC AAC     1202
   Phe Val Ala Pro Leu Tyr Trp Asp Val Tyr Ala Asn Leu Gly Phe Asn
           385                 390                 395
   TCA GCT AGA ATT TGT TTT ACC TGT CAC AAT TTT GAA TAT CAA GGA ATC     1250
   Ser Ala Arg Ile Cys Phe Thr Cys His Asn Phe Glu Tyr Gln Gly Ile
       400                 405                 410
   GCT CCA GCT CAG GAC TTA GCA TAT TGT GGT CTT GAT GTT GAT CAC CTG     1298
   Ala Pro Ala Gln Asp Leu Ala Tyr Cys Gly Leu Asp Val Asp His Leu
   415                 420                 425                 430
   GAT AGA CCA GAC AGA ATG CGG GAT AAT TCA CAT GGC AGA ATA AAT GTT     1346
   Asp Arg Pro Asp Arg Met Arg Asp Asn Ser His Gly Arg Ile Asn Val
                   435                 440                 445
   GTT AAG GGT GCA GTT GTA TAT TCC AAC ATT GTG ACA ACT GTA TCA CCA      1394
   Val Lys Gly Ala Val Val Tyr Ser Asn Ile Val Thr Thr Val Ser Pro
               450                 455                 460
   ACA TAT GCA CAA GAG GTT CGC TCA GAG GGT GGG CGT GGG CTC CAA GAT      1442
   Thr Tyr Ala Gln Glu Val Arg Ser Glu Gly Gly Arg Gly Leu Gln Asp
           465                 470                 475
   ACA CTC AAA GTG CAC TCC AAG AAA TTT GTT GGA ATA CTT AAT GGC ATT      1490
   Thr Leu Lys Val His Ser Lys Lys Phe Val Gly Ile Leu Asn Gly Ile
       480                 485                 490
   GAC ACA GAT ACT TGG AAT CCG TCT ACG GAT AGG TTT CTC AAG GTT CAA      1538
   Asp Thr Asp Thr Trp Asn Pro Ser Thr Asp Arg Phe Leu Lys Val Gln
   495                 500                 505                 510
   TAC AGT GCT AAT GAT CTA TAT GGA AAG TCA GCA AAC AAA GCA GCT CTT      1586
   Tyr Ser Ala Asn Asp Leu Tyr Gly Lys Ser Ala Asn Lys Ala Ala Leu
                   515                 520                 525
   AGG AAG CAG TTG AAG CTT GCT TCC ACA CAA GCT TCT CAA CCA TTA GTT      1634
   Arg Lys Gln Leu Lys Leu Ala Ser Thr Gln Ala Ser Gln Pro Leu Val
               530                 535                 540
   GGT TGC ATT ACG AGG CTA GTT CCT CAA AAG GGT GTA CAT CTC ATC AGG      1682
   Gly Cys Ile Thr Arg Leu Val Pro Gln Lys Gly Val His Leu Ile Arg
           545                 550                 555
   CAT GCA ATA TAT AAA ATA ACT GAG TTG GGT GGT CAA TTT GTT CTG CTG      1730
   His Ala Ile Tyr Lys Ile Thr Glu Leu Gly Gly Gln Phe Val Leu Leu
       560                 565                 570
   GGT TCA AGT CCA GTA CAG CAT ATC CAG AGA GAG TTC GAG GGT ATT GCG      1778
   Gly Ser Ser Pro Val Gln His Ile Gln Arg Glu Phe Glu Gly Ile Ala
   575                 580                 585                 590
   GAC CAA TTT CAG AAC AAC AAC AAT GTC AGG CTG CTT TTG AAG TAT GAT      1826
   Asp Gln Phe Gln Asn Asn Asn Asn Val Arg Leu Leu Leu Lys Tyr Asp
                   595                 600                 605
   GAT GCT CTG GCA CAT ATG ATC TTT GCA GCA TCA GAC ATG TTC ATT GTT      1874
   Asp Ala Leu Ala His Met Ile Phe Ala Ala Ser Asp Met Phe Ile Val
               610                 615                 620
   CCT TCT ATG TTT GAA CCA TGT GGC CTC ACT CAG ATG GTA GCT ATG CGA      1922
   Pro Ser Met Phe Glu Pro Cys Gly Leu Thr Gln Met Val Ala Met Arg
           625                 630                 635
   TAT GGT TCT GTG CCA GTT GTT CGG AGA ACC GGC GGT TTG AAT GAC AGT      1970
   Tyr Gly Ser Val Pro Val Val Arg Arg Thr Gly Gly Leu Asn Asp Ser
       640                 645                 650
   GTC TTC GAT TTG GAC GAT GAA ACG ATA CCC ATG GAG GTG CGA AAT GGC      2018
   Val Phe Asp Leu Asp Asp Glu Thr Ile Pro Met Glu Val Arg Asn Gly
   655                 660                 665                 670
   TTC ACC TTT TTG AAG GCT GAT GAG CAG GAT TTT GGT AAT GCA CTG GAA      2066
   Phe Thr Phe Leu Lys Ala Asp Glu Gln Asp Phe Gly Asn Ala Leu Glu
                   675                 680                 685
   AGA GCT TTC AAC TAC TAC CAC AGA AAA CCT GAA GTT TGG AAA CAG TTG      2114
   Arg Ala Phe Asn Tyr Tyr His Arg Lys Pro Glu Val Trp Lys Gln Leu
               690                 695                 700
   GTG CAG AAA GAC ATG AAG ATA GAT TTC AGC TGG GAT ACT TCA GTT TCT      2162
   Val Gln Lys Asp Met Lys Ile Asp Phe Ser Trp Asp Thr Ser Val Ser
           705                 710                 715
   CAA TAC GAA GAA ATC TAT CAG AAA ACA GCC ACT CGA GCC AGG GCA GCG      2210
   Gln Tyr Glu Glu Ile Tyr Gln Lys Thr Ala Thr Arg Ala Arg Ala Ala
       720                 725                 730
   GCA TAAACAGCAG AGACATTGAG ACAGTTCCCT GCTGTCTCCA TGAAGTCTCC           2263
   Ala
   735
   TAGATGCTGT GCTTAACCGT ATGGTAAAGA AATATGGTCT GTATCAGCTC AGAATTAAGC    2323
   ATCTGCCGAG GAAGCGCGGT GCATCCGGAC TCGGGTGTAC AAGGGGCGAC GTGGCGTTAC    2383
   GTGCAGTCCC CAACGAAGCA AAGAGACAGA AGTACAGCTG TACAGAACGG ATATCTTGTG    2443
   AAGCACACAT TGGGATCAGG ACGTTTGGTG CTGCAGCTAC TTTCGGTGCA GAAGCACATA    2503
   TATACGAGAC CTGCCAGGGC GAGCAAATAC CCAGTTATAC ACGCGATTGC TCAGCTCTAT    2563
   CAAGCTGTGA ATTGAAAGAT TTCTATAGTG TATTCACGCG ACGTTTTCAT AAACTAGTGT    2623
   GAGTTATGTA CTCTGACCAA AAAAAAAAA                                      2652
(2) ANGABEN ZU SEQ ID NO: 2:
   (i) SEQUENZKENNZEICHEN:
      (A) LÄNGE: 735 Aminosäuren
      (B) ART: Aminosäure
      (D) TOPOLOGIE: linear
   (ii) ART DES MOLEKÜLS: Protein

   (xi) SEQUENZBESCHREIBUNG: SEQ ID NO: 2:
   His Glu Asn Phe Leu Lys Gly Lys Leu Ile Glu Ile Thr Glu Thr Glu
   1 5 10 15
   Glu Ser Leu Phe Lys Leu Glu Lys Glu Cys Ala Leu Leu Asn Ala Ser
   20 25 30
   Leu Arg Glu Leu Glu Cys Thr Ser Thr Ser Ala Gln Ser Asp Val Leu
   35 40 45
   Lys Leu Gly Pro Leu Gln Gln Asp Ala Trp Trp Glu Lys Val Glu Asn
   50 55 60
   Leu Glu Asp Leu Leu Asp Ser Thr Ala Asn Gln Val Glu His Ala Ser
    65                  70                  75                  80
   Leu Thr Leu Asp Gly Tyr Arg Asp Phe Gln Asp Lys Val Asp Lys Leu
                    85                  90                  95
   Lys Ala Ser Leu Gly Thr Thr Asn Val Ser Glu Phe Cys Leu Tyr Leu
               100                 105                 110
   Val Asp Ile Leu Gln Gln Arg Val Lys Ser Val Glu Glu Arg Phe Gln
           115                 120                 125
   Ala Cys Asn His Glu Met His Ser Gln Ile Glu Leu Tyr Glu His Ser
       130                 135                 140
   Ile Val Glu Phe His Gly Thr Leu Ser Lys Leu Ile Asn Glu Ser Glu
   145                 150                 155                 160
   Lys Lys Ser Met Glu His Tyr Ala Glu Gly Met Pro Ser Glu Phe Trp
                   165                 170                 175
   Ser Arg Ile Ser Leu Leu Ile Asp Gly Trp Ser Leu Glu Lys Lys Ile
               180                 185                 190
   Ser Ile Asn Asp Ala Ser Met Leu Arg Glu Met Ala Trp Lys Arg Asp
           195                 200                 205
   Asn Arg Leu Arg Glu Ala Tyr Leu Ser Ser Arg Gly Met Glu Glu Arg
       210                 215                 220
   Glu Leu Ile Asp Ser Phe Leu Lys Met Ala Leu Pro Gly Thr Ser Ser
   225                 230                 235                 240
   Gly Leu His Ile Val His Ile Ala Ala Glu Met Ala Pro Val Ala Lys
                   245                 250                 255
   Val Gly Gly Leu Ala Asp Val Ile Ser Gly Leu Gly Lys Ala Leu Gln
               260                 265                 270
   Lys Lys Gly His Leu Val Glu Ile Ile Leu Pro Lys Tyr Asp Cys Met
           275                 280                 285
   Gln His Asn Gln Ile Asn Asn Leu Lys Val Leu Asp Val Val Val Lys
       290                 295                 300
   Ser Tyr Phe Glu Gly Asn Met Phe Ala Asn Lys Ile Trp Thr Gly Thr
   305                 310                 315                 320
   Val Glu Gly Leu Pro Val Tyr Phe Ile Glu Pro Gln His Pro Gly Lys
                   325                 330                 335
   Phe Phe Trp Arg Ala Gln Tyr Tyr Gly Glu His Asp Asp Phe Lys Arg
               340                 345                 350
   Phe Ser Tyr Phe Ser Arg Val Ala Leu Glu Leu Leu Tyr Gln Ser Gly
           355                 360                 365
   Lys Lys Val Asp Ile Ile His Cys His Asp Trp Gln Thr Ala Phe Val
   370 375 380
   Ala Pro Leu Tyr Trp Asp Val Tyr Ala Asn Leu Gly Phe Asn Ser Ala
   385 390 395 400
   Arg Ile Cys Phe Thr Cys His Asn Phe Glu Tyr Gln Gly Ile Ala Pro
   405 410 415
   Ala Gln Asp Leu Ala Tyr Cys Gly Leu Asp Val Asp His Leu Asp Arg
   420 425 430
   Pro Asp Arg Met Arg Asp Asn Ser His Gly Arg Ile Asn Val Val Lys
   435 440 445
   Gly Ala Val Val Tyr Ser Asn Ile Val Thr Thr Val Ser Pro Thr Tyr
   450 455 460
   Ala Gln Glu Val Arg Ser Glu Gly Gly Arg Gly Leu Gln Asp Thr Leu
   465 470 475 480
   Lys Val His Ser Lys Lys Phe Val Gly Ile Leu Asn Gly Ile Asp Thr
   485 490 495
   Asp Thr Trp Asn Pro Ser Thr Asp Arg Phe Leu Lys Val Gln Tyr Ser
   500 505 510
   Ala Asn Asp Leu Tyr Gly Lys Ser Ala Asn Lys Ala Ala Leu Arg Lys
   515 520 525
   Gln Leu Lys Leu Ala Ser Thr Gln Ala Ser Gln Pro Leu Val Gly Cys
   530 535 540
   Ile Thr Arg Leu Val Pro Gln Lys Gly Val His Leu Ile Arg His Ala
   545 550 555 560
   Ile Tyr Lys Ile Thr Glu Leu Gly Gly Gln Phe Val Leu Leu Gly Ser
   565 570 575
   Ser Pro Val Gln His Ile Gln Arg Glu Phe Glu Gly Ile Ala Asp Gln
   580 585 590
   Phe Gln Asn Asn Asn Asn Val Arg Leu Leu Leu Lys Tyr Asp Asp Ala
   595 600 605
   Leu Ala His Met Ile Phe Ala Ala Ser Asp Met Phe Ile Val Pro Ser
   610 615 620
   Met Phe Glu Pro Cys Gly Leu Thr Gln Met Val Ala Met Arg Tyr Gly
   625 630 635 640
   Ser Val Pro Val Val Arg Arg Thr Gly Gly Leu Asn Asp Ser Val Phe
   645 650 655
   Asp Leu Asp Asp Glu Thr Ile Pro Met Glu Val Arg Asn Gly Phe Thr
   660 665 670
   Phe Leu Lys Ala Asp Glu Gln Asp Phe Gly Asn Ala Leu Glu Arg Ala
           675                 680                 685
   Phe Asn Tyr Tyr His Arg Lys Pro Glu Val Trp Lys Gln Leu Val Gln
       690                 695                 700
   Lys Asp Met Lys Ile Asp Phe Ser Trp Asp Thr Ser Val Ser Gln Tyr
   705                 710                 715                 720
   Glu Glu Ile Tyr Gln Lys Thr Ala Thr Arg Ala Arg Ala Ala Ala
                   725                 730                 735